# EUROPÄISCHE PATENTANMELDUNG

(11) **EP 3 834 701 A1**
(43) Veröffentlichungstag der Anmeldung: **16.06.2021**
(21) Anmeldenummer: 20212736.1
(22) Anmeldetag: 09.12.2020
(51) Int. Cl.: A61B 1/00, A61B 1/06, A61B 1/04, G02B 21/16, G02B 23/24, A61B 5/00, G01J 3/44, G01N 21/64, G02B 5/20

(54) **MEDIZINISCHE BILDGEBUNGSVORRICHTUNG**

(30) Priorität: 13.12.2019 DE 102019134314; 02.03.2020 DE 102020105458
(71) Anmelder: Karl Storz SE & Co. KG, 78532 Tuttlingen (DE)
(72) Erfinder: Baumann, Harald, 78532 Tuttlingen (DE); Baumann, Christin, 78532 Tuttlingen (DE)

(57) **Zusammenfassung**

Die Erfindung geht aus von einer medizinischen Bildgebungsvorrichtung, insbesondere einer endoskopischen Bildgebungsvorrichtung, mit wenigstens einer Bilderfassungseinheit (10), welche wenigstens eine Bilderfassungssensorik (12) und wenigstens eine optische Filtereinheit (16) aufweist, welche wenigstens einen Spektralfilter (18) umfasst, welcher der Bilderfassungssensorik (12) zugeordnet und in Blickrichtung (20) der Bilderfassungssensorik (12) betrachtet vor dieser angeordnet ist.

Es wird vorgeschlagen, dass der Spektralfilter (18) in einem ersten Betriebszustand dazu ausgebildet ist, Licht gemäß eines ersten spektralen Transmissionsbereichs (22) zur Bilderfassungssensorik (12) zu transmittieren und in einem zweiten Betriebszustand dazu ausgebildet ist, Licht gemäß eines zweiten spektralen Transmissionsbereichs (24), welcher von dem ersten spektralen Transmissionsbereich (22) zumindest teilweise verschieden ist, zur Bilderfassungssensorik (12) zu transmittieren.

## Beschreibung

### Stand der Technik

Die Erfindung betrifft eine medizinische Bildgebungsvorrichtung nach dem Oberbegriff des Anspruchs 1, sowie ein Verfahren zum Betrieb einer medizinischen Bildgebungsvorrichtung nach dem Oberbegriff des Anspruchs 23.

Aus der EP1327414B1 ist bereits eine Vorrichtung zur bildgebenden Diagnose von Gewebe bekannt, wobei ein Transmissionsgrad eines Beleuchtungslichts mittels eines variablen Spektralfilters einstellbar ist.

Die Aufgabe der Erfindung besteht insbesondere darin, eine gattungsgemäße Vorrichtung mit verbesserten Eigenschaften hinsichtlich einer Effizienz, insbesondere in Bezug auf eine Effizienz einer Bildgebung und/oder einer Bauraum- bzw. Bauteileffizienz, bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer medizinischen Bildgebungsvorrichtung, insbesondere einer endoskopischen Bildgebungsvorrichtung, mit wenigstens einer Bilderfassungseinheit, welche wenigstens eine Bilderfassungssensorik und wenigstens eine optische Filtereinheit aufweist, die wenigstens einen Spektralfilter umfasst, welcher der Bilderfassungssensorik zugeordnet und in Blickrichtung der Bilderfassungssensorik betrachtet vor dieser angeordnet ist.

Es wird vorgeschlagen, dass der Spektralfilter in wenigstens einem ersten Betriebszustand dazu ausgebildet ist, Licht gemäß eines ersten spektralen Transmissionsbereichs zur Bilderfassungssensorik zu transmittieren und in wenigstens einem zweiten Betriebszustand dazu ausgebildet ist, Licht gemäß eines zweiten spektralen Transmissionsbereichs, welcher von dem ersten spektralen Transmissionsbereich zumindest teilweise verschieden ist, zur Bilderfassungssensorik zu transmittieren. Hierdurch kann insbesondere eine medizinische Bilderfassungsvorrichtung mit einer verbesserten Effizienz bereitgestellt werden. Insbesondere kann eine Effizienz einer Bildgebung verbessert werden, da insbesondere spektrale Bildgebungsanteile durch den mittels des ersten spektralen Transmissionsbereich und des zweiten spektralen Transmissionsbereich unterschieden werden können. Zudem kann vorteilhaft eine Bauraum- bzw. Bauteileffizienz der medizinischen Bildgebungsvorrichtung verbessert werden, da anstelle von je einem Spektralfilter je Transmissionsbereich, ein einzelner Spektralfilter genutzt werden kann, dessen Stellungsabhängigkeit relativ zum Bilderfassungssensorik die Bereitstellung sowohl eines ersten spektralen Transmissionsberiech als auch eines zweiten spektralen Transmissionsbereich ermöglicht, wodurch als Bauteile zusätzlich Spektralfilter und ein Bauraum für deren Anordnung eingespart werden können.

Die medizinische Bildgebungsvorrichtung ist insbesondere zu einer multispektralen Bildgebung und vorzugsweise einer hyperspektralen Bildgebung eingerichtet. Unter einer "multispektralen Bildgebung" soll dabei insbesondere eine Bildgebung verstanden werden, bei welchen wenigstens drei Spektralbänder voneinander unabhängig erfassbar sind. Unter einer "hyperspektralen Bildgebung" soll dabei insbesondere eine Bildgebung verstanden werden, bei welcher wenigstens zwanzig Spektralbänder voneinander unabhängig erfassbar sind. Besonders bevorzugt eignet sich die vorliegende medizinische Bildgebungsvorrichtung zu einer multispektralen Bildgebung und vorzugsweise einer hyperspektralen Bildgebung gemäß eines zeitlich aufgelösten Schnappschussverfahrens, bei welchem die spektralen Bänder zeitlich versetzt mit nur einem einzigen Detektor-Output erfasst werden. Unter einer "medizinischen Bildgebungsvorrichtung" soll insbesondere ein, vorzugsweise funktionsfähiger Bestandteil, insbesondere eine Unterbaugruppe und/oder eine Konstruktions- und/oder eine Funktionskomponente eines medizinischen Bildgebungsgeräts und/oder eines medizinischen Bildgebungssystems verstanden werden. Vorzugsweise kann die medizinische Bildgebungsvorrichtung das medizinische Bildgebungsgerät und/oder das medizinische Bildgebungssystem zumindest teilweise, vorzugsweise zumindest zu einem Großteil und besonders bevorzugt vollständig ausbilden. Bei der medizinischen Bildgebungsvorrichtung kann es sich um eine mikroskopische, makroskopische und/oder exoskopische Bildgebungsvorrichtung handeln, welche beispielsweise als ein Bildgebungsgerät, zumindest teilweise und vorzugsweise zumindest zu einem Großteil ein Mikroskop, ein Makroskop und/oder ein Exoskop und/oder als ein Bildgebungssystem, zumindest teilweise ein Mikroskop-, ein Makroskop- und/oder ein Exoskopsystem ausbildet. Bevorzugt ist die medizinische Bildgebungsvorrichtung als eine endoskopische Bildgebungsvorrichtung ausgebildet, welche ein insbesondere zumindest teilweise und vorzugsweise zumindest zu einem Großteil ein Endoskop und/oder ein Endoskopsystem ausbildet. Die endoskopische Bildgebungsvorrichtung ist insbesondere dazu ausgebildet, zumindest teilweise und vorzugsweise zumindest zu einem Großteil in eine künstliche, wie beispielsweise ein Gehäuse, und/oder natürliche Kavität, wie beispielsweise einen Hohlraum in einem Körperorgan oder im Gewebe, eingeführt zu werden, um diese zu begutachten. Unter "ausgebildet" soll insbesondere speziell programmiert, ausgelegt und/oder ausgestattet verstanden werden. Darunter, dass ein Objekt zu einer bestimmten Funktion ausgebildet ist, soll insbesondere verstanden werden, dass das Objekt diese bestimmte Funktion in zumindest einem Anwendungs- und/oder Betriebszustand erfüllt und/oder ausführt. Unter dem Ausdruck "zumindest zu einem Großteil" soll dabei insbesondere zumindest zu 55 %, vorzugsweiser zumindest zu 65 %, bevorzugt zumindest zu 75 %, besonders bevorzugt zumindest zu 85 % und ganz besonders bevorzugt zumindest zu 95 % sowie vorteilhaft vollständig verstanden werden und zwar insbesondere mit Bezug auf ein Volumen und/oder eine Masse eines Objekts.

Die medizinische Bilderfassungsvorrichtung weist wenigstens einen proximalen Abschnitt, einen distalen Abschnitt und/oder einen Zwischenabschnitt auf. Der distale Abschnitt ist insbesondere dazu ausgebildet, in einem Betriebszustand in einer zu untersuchenden Kavität eingeführt zu werden. Der proximale Abschnitt ist insbesondere dazu ausgebildet, in einem Betriebszustand außerhalb der zu untersuchenden Kavität angeordnet zu sein. Unter "distal" soll insbesondere bei einer Bedienung einem Patienten zugewandt und/oder einem Bediener abgewandt verstanden werden. Insbesondere ist proximal das Gegenteil von distal. Unter "proximal" soll insbesondere bei einer Bedienung einem Patienten abgewandt und/oder einem Bediener zugewandt verstanden werden. Die medizinische Bilderfassungsvorrichtung weist insbesondere zumindest einen, vorzugsweise flexiblen Schaft auf. Der Schaft ist als ein längliches Objekt ausgebildet. Ferner bildet der Schaft zumindest teilweise und vorzugsweise zumindest zu einem Großteil den distalen Abschnitt aus. Unter einem "länglichen Objekt" soll insbesondere ein Objekt verstanden werden, dessen Haupterstreckung zumindest um einen Faktor fünf, vorzugsweise zumindest um einen Faktor zehn und besonders bevorzugt zumindest um einen Faktor zwanzig größer ist als eine größte Erstreckung des Objekts senkrecht zu dessen Haupterstreckung, also insbesondere einem Durchmesser des Objekts. Unter einer "Haupterstreckung" eines Objekts, soll insbesondere dessen längste Erstreckung entlang dessen Haupterstreckungsrichtung verstanden werden. Unter einer "Haupterstreckungsrichtung" eines Bauteils soll dabei insbesondere eine Richtung verstanden werden, welche parallel zu einer längsten Kante eines kleinsten gedachten Quaders verläuft, welcher das Bauteil gerade noch vollständig umschließt.

Unter einer "Bilderfassungseinheit" soll insbesondere eine Einheit verstanden werden, welche zu einer Bilderfassung ausgebildet ist und dazu zumindest eine Bilderfassungssensorik umfasst. Die Bilderfassungseinheit ist vorzugsweise zumindest teilweise und vorzugsweise zumindest zu einem Großteil im Bereich des proximalen Abschnitts angeordnet und/oder diesen ausbildet. Alternativ ist denkbar, dass die Bilderfassungseinheit zumindest teilweise und vorzugsweise zumindest zu einem Großteil im distalen Abschnitt angeordnet sein kann und/oder diesen ausbildet. Ferner könnte die Bilderfassungseinheit zumindest teilweise auf den proximalen Abschnitt und den distalen Abschnitt verteilt angeordnet sein. Die Bilderfassungssensorik weist insbesondere zumindest einen Bildsensor auf. Ferner kann die Bilderfassungssensorik auch über zumindest zwei und vorzugsweise mehrere Bildsensoren verfügen, welche beispielsweise in Blickrichtung der Bilderfassungssensorik hintereinander angeordnet sein können. Ferner können die zwei und vorzugsweise mehreren Bilderfassungssensoren über voneinander verschieden ausgebildete spektrale Erfassungsempfindlichkeiten verfügen, sodass beispielsweise ein erster Sensor im Vergleich in einen roten Spektralbereich, ein zweiter Sensor im Vergleich in einen blauen Spektralbereich und ein dritter Sensor im Vergleich einen grünen Spektralbereich besonders empfindlich ist. Der Bildsensor kann in etwa als ein CCD-Sensor ausgebildet sein. Bevorzugt ist der Bildsensor als ein CMOS-Sensor ausgebildet. Unter einer "Blickrichtung" der Bilderfassungssensorik soll insbesondere eine Richtung verstanden werden, welche von einer aktiven Seite der Bilderfassungssensorik weg zeigt und vorzugsweise zumindest im Wesentlichen parallel zur optischen Achse der Bilderfassungssensorik ist. Unter "zumindest im Wesentlichen parallel" soll hier insbesondere eine Ausrichtung einer Richtung relativ zu einer Bezugsrichtung, insbesondere in einer Ebene verstanden werden, wobei die Richtung und die Bezugsrichtung einen Winkel von 0° insbesondere unter Berücksichtigung einer maximalen Abweichung von kleiner als 8°, vorteilhaft von kleiner als 5° und besonders vorteilhaft von kleiner als 2° einschließt. Ferner könnte die Blickrichtung vorzugsweise zumindest im Wesentlichen senkrecht auf einer Haupterstreckungseben der Bilderfassungssensorik stehen. Unter "zumindest im Wesentlichen senkrecht" soll hier insbesondere eine Ausrichtung einer Richtung relativ zu einer Bezugsrichtung, insbesondere in einer Ebene, verstanden werden, wobei die Richtung und die Bezugsrichtung einen Winkel von 90°, insbesondere unter Berücksichtigung einer maximalen Abweichung von kleiner als 8°, vorteilhaft von kleiner als 5° und besonders vorteilhaft von kleiner als 2° einschließt. Unter einer "Haupterstreckungsebene" eines Objekts soll insbesondere eine Ebene verstanden werden, welche parallel zu einer größten Seitenfläche eines kleinsten gedachten Quaders ist, welcher das Objekt gerade noch vollständig umschließt, und insbesondere durch den Mittelpunkt des Quaders verläuft. Unter einer "optischen Filtereinheit" soll insbesondere eine Einheit verstanden werden, welche zu einem Filtern optischer Spektralbereiche vorgesehen ist und dazu zumindest einen Spektralfilter umfasst. Der Spektralfilter ist insbesondere als ein, insbesondere Bandpassfilter ausgebildet. Ferner könnte der Spektralfilter als ein Polarisationsfilter ausgebildet sein und zwar vorzugsweise als ein Polarisation Bandpassfilter. Unter einem "Transmissionsbereich eines Spektralfilters" soll insbesondere ein Spektralbereich des Spektralfilters verstanden werden, in welchem dieser zumindest einen Großteil eines dem Spektrum des Transmissionsbereichs entsprechenden einfallenden Lichts passieren lässt. Weitere Spektralbereiche des Spektralfilters, welche von dem Transmissionsbereich verschieden sind sollen als Blockierungsbereiche verstanden werden. Unter einem "Off-Band-Bereich eines Spektralfilters" soll insbesondere ein Spektralbereich des Spektralfilters verstanden werden, in welchem dieser höchstens einen Bruchteil eines dem Spektrum des Off-Band-Bereichs entsprechenden einfallenden Lichts passieren lässt. Unter einem "Bruchteil" soll in diesem Zusammenhang insbesondere weniger als 10 %, vorzugsweise weniger als 5 % und besonders bevorzugt weniger als 1 % verstanden werden. Der Spektralfilter ist insbesondere als ein Bandpassfilter ausgebildet. Der Spektralfilter weist vorzugsweise in einem Betriebszustand nur einen einzigen Transmissionsbereich auf. Der erste Transmissionsbereich und der weitere erste Transmissionsbereich sind insbesondere nie gleichzeitig in einem selben Betriebszustand des Spektralfilters vorhanden.

Beispielsweise wird durch eine Überführung des Spektralfilters von dem ersten Betriebszustand in den zweiten Betriebszustand der erste Transmissionsbereich in den zweiten Transmissionsbereich überführt und/oder umgekehrt. Ferner ist denkbar, dass der Spektralfilter weitere Betriebszustände, insbesondere Zwischenzustände, annehmen kann und derart Licht weiterer Transmissionsbereiche transmittierbar ist. Beispielsweise könnten Betriebszustände des Spektralfilters kontinuierlich veränderbar sein, wodurch kontinuierliche verschiedene Transmissionsbereiche erzielbar sind. Der erste Transmissionsbereich und der zweite Transmissionsbereich sind insbesondere spektral zueinander verschoben. Ein Grad der Verschiebung der Transmissionsbereiche ist dabei insbesondere korreliert mit dem Betriebszustand des Spektralfilters.

Ferner wird vorgeschlagen, dass der erste Betriebszustand definiert ist als eine erste Stellung des Spektralfilters relativ zur Bilderfassungssensorik und der zweite Betriebszustand definiert ist als eine zweite Stellung des Spektralfilters relativ zur Bilderfassungsssensorik. Es kann vorteilhaft ein Aufbau des Spektralfilters vereinfacht werden. Ferner kann eine Vielfalt verschiedener Transmissionsbereiche in Abhängigkeit von der Stellung des Spektralfitlers erzielt werden. Unter einer "Stellung des Spektralfilters relativ zur Bilderfassungssensorik" soll insbesondere eine Lage und/oder Orientierung im Raum des Spektralfilters und des Bilderfassungssensors relativ zueinander verstanden werden. Insbesondere sind die erste Stellung und die zweite Stellung voneinander verschiedene Stellungen des Spektralfilters und des Bilderfassungssensors relativ zueinander. Die erste Stellung und die zweite Stellung unterscheiden sich in etwa durch wenigstens einen Winkel zwischen dem Spektralfilter und der Bilderfassungssensorik. Der Winkel ist insbesondere von der Haupterstreckungsebene des Spektralfilters und einer Haupterstreckungsebene des Bilderfassungssensorik aufgespannt. Der Winkel beträgt insbesondere weniger als 180°, vorzugsweise weniger als 90° und besonders bevorzugt weniger als 45°. Der Winkel beträgt mehr als 0°, vorzugsweise mehr als 5° und besonders bevorzugt mehr als 10°. Im vorliegenden Fall beträgt der Winkel im Wesentlichen 30°. Unter "im Wesentlichen" soll insbesondere der Wert unter Berücksichtigung einer maximale Abweichung von höchstens 10 %, vorzugsweise von höchstens 5 % und besonders bevorzugt von höchstens 2 % oder aber auch genau der Wert selbst verstanden werden. In der ersten Stellung ist der Spektralfilter zumindest im Wesentlichen senkrecht zur optischen Achse angeordnet. Ferner ist insbesondere in der ersten Stellung die Bilderfassungssensor zumindest im Wesentlichen senkrecht zur optischen Achse angeordnet. Es ist denkbar, dass insbesondere abhängig von einem Gesamtaufbau, in der ersten Stellung der Spektralfilter und der Bilderfassungssensorik zumindest im Wesentlichen parallel zueinander angeordnet sind. Vorzugsweise ist in der ersten Stellung eine Haupterstreckungsebene des Spektralfilters zumindest im Wesentlichen parallel zu einer Haupterstreckungsebene der Bilderfassungssensorik. In der zweiten Stellung ist der Spektralfilter winklig zur optischen Achse angeordnet. Es ist denkbar, dass insbesondere abhängig von einem Gesamtaufbau, in der ersten Stellung der Spektralfilter und der Bilderfassungssensorik winklig zueinander angeordnet sind. Unter winklig soll insbesondere verschieden von "zumindest im Wesentlichen parallel" verstanden werden. In der zweiten Stellung könnte der Winkel vorzugsweise zwischen der Haupterstreckungsebene des Spektralfilters und der Haupterstreckungsebene der Bilderfassungssensorik. Ferner ist denkbar, dass der Spektralfilter und der Bilderfassungssensor in weiteren Stellungen zueinander angeordnet werden können und derart Licht weitere Transmissionsbereiche transmittierbar ist. Beispielsweise könnte eine Stellung des Spektralfilters relativ zu dem Bilderfassungssensor kontinuierlich veränderbar sein, wodurch kontinuierliche verschiedene Transmissionsbereiche erzielbar sind. Der erste Transmissionsbereich und der zweite Transmissionsbereich sind insbesondere spektral zueinander verschoben. Ein Grad der Verschiebung der Transmissionsbereiche ist dabei insbesondere korreliert mit einem Winkel zwischen der ersten Stellung und der zweiten Stellung der Spektralfilter, wobei der Grad der Verschiebung vorzugsweise mit zunehmendem Winkel ebenfalls zunimmt.

Es wird des Weiteren vorgeschlagen, dass der erste spektrale Transmissionsbereich eine erste Filterkante und der zweite spektrale Transmissionsbereich eine zweite Filterkante aufweist, welche relativ zur ersten Filterkante spektral verschoben ist. Es kann vorteilhaft eine Effizienz der Bildgebung weiter verbessert werden. Insbesondere kann eine spektrale Trennung der Transmissionsbereiche verbessert werden. Weiter vorteilhaft kann eine scharfe Trennung der Transmissionsbereiche erzielt werden. Unter einer "Filterkante" soll insbesondere ein Übergangsbereich zwischen einem Off-Band-Bereich und einem Transmissionsbereich verstanden werden. Insbesondere handelt es sich wenigstens bei der ersten Filterkante und/oder der zweiten Filterkante um eine steigende Filterkante. Unter einer "steigenden Filterkante" soll insbesondre eine Filterkante verstanden werden, welche den Übergang von einem Off-Band-Bereich hin zu einem Transmissionsbereich beschreibt. Insbesondere ist ein Wert einer Wellenlänge der zweiten Filterkante kleiner als ein Wert einer Wellenlänge der ersten Filterkante. Der Wert der zweiten Filterkante ist insbesondere zumindest um 1 %, vorzugsweise zumindest um 10 % und besonders bevorzugt zumindest um 20 % verschieden von dem Wert der ersten Filterkante und insbesondere um diesen Faktor kleiner als der Wert der ersten Filterkante.

Weiterhin wird vorgeschlagen, dass den ersten spektralen Transmissionsbereich eine erste Zentralwellenlänge und der zweite spektrale Transmissionsbereich eine zweite Zentralwellenlänge aufweist, welche relativ zur ersten Zentralwellenlänge spektral verschoben ist. Es kann vorteilhaft eine Effizienz der Bildgebung weiter verbessert werden. Insbesondere kann eine spektrale Trennung der Transmissionsbereiche verbessert werden. Weiter vorteilhaft können die Transmissionsbereiche auf vorgesehene Wellenlängenbereiche abgestimmt werden. Unter einer "Zentralwellenlänge" soll insbesondere der Mittelwert des spektralen Transmissionsbereichs verstanden werden. Insbesondere ist ein Wert einer Wellenlänge der zweiten Zentralwellenlänge kleiner als ein Wert einer Wellenlänge der ersten Zentralwellenlänge. Der Wert der zweiten Zentralwellenlänge ist vorzugsweise zumindest um 1 %, vorzugsweise zumindest um 10 % und besonders bevorzugt zumindest um 20 % verschieden von dem Wert der ersten Zentralwellenlänge und insbesondere um diesen Faktor kleiner als der Wert der ersten Zentralwellenlänge.

Es wird außerdem vorgeschlagen, dass der erste spektrale Transmissionsbereich eine erste Bandbreite und der zweite spektrale Transmissionsbereich eine zweite Bandbreite aufweist, welche zumindest zu einem Großteil der ersten Bandbreite entspricht. Es kann vorteilhaft eine Effizienz der Bildgebung weiter verbessert werden. Insbesondere kann eine spektrale Trennung der Transmissionsbereiche verbessert werden. Weiter vorteilhaft können die Transmissionsbereiche auf vorgesehene Wellenlängenbereiche abgestimmt werden. Unter einer "Bandbreite" soll insbesondere eine Breite des spektralen Transmissionsbereichs verstanden werden. Unter "zu einem Großteil entsprechend" soll insbesondere verstanden werden, dass eine von dem ersten Transmissionsbereich eingeschlossene Fläche zumindest zu einem Großteil der von dem zweiten Transmissionsberiecht eingeschlossenen Fläche entspricht und sich vorzugsweise zumindest zu einem Großteil mit dieser überschneidet. Insbesondere ist ein Wert einer Wellenlänge der zweiten Bandbreite kleiner als ein Wert einer Wellenlänge der ersten Bandbreite. Der Wert der zweiten Bandbreite ist insbesondere zumindest um 1 %, vorzugsweise zumindest um 10 % und besonders bevorzugt zumindest um 20 % verschieden von dem Wert der ersten Bandbreite und insbesondere um diesen Fakor kleiner als der Wert der ersten Bandbreite.

Es wird ferner vorgeschlagen, dass der Spektralfilter als ein Interferenzfilter ausgebildet ist. Es kann vorteilhaft eine Effizienz der Bildgebung weiter verbessert werden. Insbesondere können Wellenlängenbereich von Transmissionsspektren maßgeschneidert auf eine Anwendungsbereich abgestimmt werden. Ferner könnte es sich bei dem Spektralfilter um einen Interferenzspiegel, einen Interferenzstrahlteiler oder dergleichen handeln. Der Interferenzfilter könnte als ein Monochromator oder aber auch ein dielektrischer Spiegel ausgebildet sein.

Ferner wird vorgeschlagen, dass die Bilderfassungseinheit dazu eingerichtet ist, den Spektralfilter von dem ersten Betriebszustand in den zweiten Betriebszustand und/oder umgekehrt zu überführen. Um den Betriebszustand des Spektralfilters, insbesondere relativ zum Bilderfassungssensorik zu verändern, könnte in etwa die Bilderfassungssensorik verstellbar sein, beispielsweise könnte die Bilderfassungseinheit dazu ein Lager aufweisen. Bevorzugt ist jedoch der Spektralfilter zur Veränderung des Betriebszustands des Spektralfilters, insbesondere relativ zur Bilderfassungssensorik, verstellbar. Alternativ könnten sowohl der Spektralfilter, als auch die Bilderfassungssensorik verstellbar sein. Besonders bevorzugt weist die Filtereinheit wenigstens ein Lager auf, welches dazu ausgebildet ist, den Spektralfilter von dem ersten Betriebszustand in den zweiten Betriebszustand und/oder umgekehrt überführbar zu lagern. Es kann vorteilhaft ein Bauraumteil bzw. eine Bauraumeffizienz verbessert werden. Bei dem Lager handelt es sich insbesondere um ein Schwenk- und/oder Kipplager. Das Lager ist insbesondere zu einem Verschwenken des Spektralfilters um eine zur Haupterstreckungsebene des Spektralfilters liegende Schwenkachse vorgesehen. Ferner könnte das Lager zu einem Verkippen des Spektralfilters um eine in der Haupterstreckungsebenen liegenden Kippachse ausgebildet sein.

In einer Ausgestaltung der Erfindung wird vorgeschlagen, dass die Filtereinheit wenigstens einen Aktor umfasst, welcher dazu ausgebildet ist, den Spektralfilter von der ersten Betriebszustand in den zweiten Betriebszustand und/oder umgekehrt zu überführen. Es kann vorteilhaft ein Bauteil bzw. eine Bauraumeffizienz verbessert werden. Insbesondere kann auf eine aufwendige Mechanik zur Verstellung des Spektralfilters verzichtet werden. Der Aktor kann insbesondere als ein Schrittmotor ausgebildet sein, wobei vorzugsweise Einzelschritte des Aktors Winkelsegmente des Winkels zwischen dem ersten Betriebszustand und zweiten Betriebszustand des Spektralfilters, insbesondere relativ zur Bilderfassungssensorik, entsprechen können. Alternativ oder zusätzlich könnte die Bilderfassungseinheit zur Verstellung der Bilderfassungssensorik einen Aktor aufweisen.

In einer bevorzugten Ausgestaltung der Erfindung wird vorgeschlagen, dass der Aktor dazu ausgebildet ist, mindestens 15 mal, vorzugsweise mindestens 30 mal und besonders bevorzugt mindestens 60 mal pro Sekunde den Spektralfilter von dem ersten Betriebszustand in den zweiten Betriebszustand des Spektralfilters und/oder umgekehrt zu überführen. Es kann vorteilhaft eine Visualisierung von Bildinformationen verbessert werden. Insbesondere können bewegte Bildinformationen aufgenommen werden, welche Beobachtungen in Echtzeit ermöglichen. Weiter vorteilhaft können in Echtzeit Bildinformationen, welche zum einen dem ersten spektralen Transmissionsberiech und zum anderen dem zweiten spektralen Transmissionsbereich entsprechen bereitgestellt werden. Dabei können diese insbesondere als separate oder überlagerte Bildinformationen bereitgestellt werden. Ferner ist der Aktor dazu ausgebildet, mindestens 15 mal, vorzugsweise mindestens 30 mal und besonders bevorzugt mindestens 60 mal pro Sekunde den Spektralfilter von dem zweiten Betriebszustand in den ersten Betriebszustand des Spektralfilters zu überführen.

Zudem wird vorgeschlagen, dass die medizinische Bildgebungsvorrichtung wenigstens eine Beleuchtungseinheit umfasst, welche zur Beleuchtung eines von der Bilderfassungseinheit zu erfassenden Objekts ausgebildet ist. Es kann vorteilhaft eine Bildgebung weiter verbessert werden. Insbesondere kann eine Beleuchtung zu untersuchender Objekte verbessert werden. Weiterhin kann eine gezielte Anregung zu untersuchender Objekte durch die bereitgestellte Beleuchtung erzielt werden. Bei dem zu erfassenden Objekt kann es sich um in einer Kavität angeordnetes Objekt handeln, wie beispielsweise Gewebe, ein Organ oder dergleichen. Zur Beleuchtung weist die Beleuchtungseinheit zumindest ein Beleuchtungselement auf. Insbesondere kann die Beleuchtungseinheit verschieden ausgebildete Beleuchtungselemente aufweisen, welche dazu ausgebildet sein können, verschiedene Beleuchtungsspektren bereitzustellen. Beispielsweise kann ein Beleuchtungselement als LED, Laser, Laserdiode, Halogenlampe, Weißlichtlampe oder dergleichen ausgebildet sein. Alternativ oder zusätzlich kann die Beleuchtungseinheit über einen Beleuchtungsfilter verfügen, welcher dazu ausgebildet ist, vorgesehene Beleuchtungsspektren, welche als Beleuchtungslicht eine Lichtquelle dienen, zu transmittieren. Das zu beleuchtende Objekt kann insbesondere das Beleuchtungsspektrum reflektieren. Ferner kann das zu beleuchtende Objekt mit spontan Licht emittierendes Material umfassen und/oder mit diesem versehen sein, welches vorzugsweise basierend auf einer Anregung durch das Beleuchtungsspektrum, welches im Wesentlichen einem Absorptionsspektrum des Materials entspricht Licht eines Emissionsspektrums emittiert.

Ferner wird vorgeschlagen, dass die Beleuchtungseinheit dazu ausgebildet ist, wenigstens ein erstes Beleuchtungslicht gemäß eines Beleuchtungsspektrums, welches zumindest zu einem Großteil außerhalb des ersten spektralen Transmissionsbereichs liegt, bereitzustellen. Es kann vorteilhaft eine Bildgebung weiter verbessert werden. Insbesondere kann eine Anregung von spontan Licht emittierendem Material durch das Beleuchtungslicht erzielt werden, ohne dass das Beleuchtungslicht ein Emissionsspektrum des Materials bei einer Bildgebung maßgeblich überlagert. Unter "einem Großteil außerhalb" soll hier insbesondere verstanden werden, dass ein Großteil einer von dem Beleuchtungsspektrum eingeschlossenen Fläche außerhalb einer von dem Transmissionsberiech eingeschlossenen Fläche liegt. Insbesondere liegt das Beleuchtungsspektrum spektral unterhalb des ersten spektralen Transmissionsbereichs.

Des Weiteren wird vorgeschlagen, dass das Beleuchtungsspektrum zumindest zu einem Großteil einem Absorptionsspektrum eines spontan Licht emittierenden Materials des zu beleuchtenden Objekts entspricht. Unter "zumindest zu einem Großteil entsprechend" soll insbesondere verstanden werden, dass eine von dem Beleuchtungsspektrum eingeschlossene Fläche zumindest zu einem Großteil einer von dem Absorptionsspektrum eingeschlossenen Fläche entspricht und vorzugsweise sich zumindest zu einem Großteil mit dieser überlagert. Bei dem spontan Licht emittierenden Material kann es sich insbesondere um ein fluoreszierendes und/oder phosphoreszierendes Material, insbesondere Farbstoff, handeln. Vorzugsweise ist das spontan Licht emittierende Material Teil von Gewebe, wie beispielsweise fluoreszierende Proteine, insbesondere Porphyrine und vorzugsweise Protoporphyrine. Ferner kann es sich bei dem Material um Fluorophore handeln, insbesondere Fluoreszin, Cyanin oder dergleichen und zwar besonders bevorzugt Indocyaningrün (ICG - Indocyanine green) und/oder OTL38.

Es wird weiterhin vorgeschlagen, dass ein Emissionsspektrum eines mittels der Beleuchtungseinheit beleuchteten spontan Licht emittierenden Materials zumindest zu einem Großteil im ersten spektralen Transmissionsbereich liegt. Es kann vorteilhaft eine Bildgebung verbessert werden. Insbesondere kann derart ein Großteil des von dem Material emittierten Lichts bei der Bildgebung berücksichtigt werden. Somit kann insbesondere in dem ersten Betriebszustand eine Autofluoreszenz- und/oder Fluoreszenzdarstellung erzielt werden. Vorzugsweise handelt es sich bei dem Emissionsspektrum um das Emissionsspektrum des autofluoreszierenden Gewebes und/oder eines Farbstoffs und zwar besonders bevorzugt Indocyaningrün.

Weiter wird vorgeschlagen, dass die Beleuchtungseinheit dazu ausgebildet ist, wenigstens ein Beleuchtungslicht mit einem Beleuchtungsspektrum, insbesondere Weißlicht bereitzustellen, welches zumindest zu einem Großteil innerhalb dem zweiten spektralen Transmissionsbereich liegt. Es kann vorteilhaft eine Bildgebung verbessert werden. Insbesondere kann derart ein Großteil des von dem Material reflektierten Lichts bei der Bildgebung berücksichtigt werden. Somit kann insbesondere in dem zweiten Betriebszustand eine Weißlichtdarstellung erzielt werden, welches auf der Reflexion und/oder der Absorption spektraler Anteile des Beleuchtungslichts durch das zu untersuchenden Objekt beruht. Unter zumindest "zu einem Großteil innerhalb" soll insbesondere verstanden, dass zumindest ein Großteil einer von Beleuchtungsspektrum eingeschlossenen Fläche innerhalb einer von dem zweiten Transmissionsbereich eingeschlossenen Fläche liegt und sich vorzugsweise mit dieser zumindest zu einem Großteil mit dieser überschneidet.

Es wird außerdem vorgeschlagen, dass ein weiteres Emissionsspektrum eines mittels der Beleuchtungseinheit beleuchteten weiteren spontan Licht emittierenden Materials zumindest zu einem Großteil außerhalb eines ersten spektralen Transmissionsbereichs liegt. Es kann vorteilhaft eine Bildgebung weiter verbessert werden, bei welcher zwischen dem Emissionsspektrum und dem weiteren Emissionsspektrum unterschieden werden kann. Unter zumindest "zu einem Großteil außerhalb" soll insbesondere verstanden, dass zumindest ein Großteil einer von Emissionsspektrum eingeschlossenen Fläche außerhalb einer von dem ersten Transmissionsbereich eingeschlossenen Fläche liegt und sich vorzugsweise mit dieser zumindest zu einem Großteil keine Überschneidung aufweist.

Zudem wird vorgeschlagen, dass ein weiteres Emissionsspektrum eines mittels der Beleuchtungseinheit beleuchteten weiteren spontan Licht emittierenden Materials zumindest zu einem Großteil innerhalb des zweiten spektralen Transmissionsbereichs liegt. Es kann vorteilhaft eine Bildgebung weiter verbessert werden, bei welcher zwischen dem Emissionsspektrum und dem weiteren Emissionsspektrum unterschieden werden kann. Unter zumindest "zu einem Großteil innerhalb" soll insbesondere verstanden, dass zumindest ein Großteil einer von dem Emissionsspektrum eingeschlossenen Fläche innerhalb einer von dem zweiten Transmissionsbereich eingeschlossenen Fläche liegt und sich vorzugsweise mit dieser zumindest zu einem Großteil mit dieser überschneidet. Vorzugsweise handelt es sich bei dem weiteren spontan Licht emittierenden Material um OTL38.

Zu einer Abbildung transmittierten Lichts kann die Filtereinheit eine Abbildungslinse aufweisen. Es wäre denkbar, dass eine solche Abbildungslinse vor oder hinter dem Spektralfilter angeordnet seien kann. Um jedoch chromatische Abbildungsfehler zu verringern und/oder zu vermeiden, wird vorgeschlagen, dass die Filtereinheit wenigstens eine schaltbare Abbildungslinse umfasst, welche zwischen Spektralfilter und Bilderfassungssensorik angeordnet ist und welche dazu ausgebildet ist, abhängig von einem Betriebszustand des Spektralfilters, ein von dem Spektralfilter transmittiertes Transmissionsspektrum auf die Bilderfassungssensorik abzubilden. Unter einer schaltbaren Abbildungslinse soll insbesondere eine Abbildungslinse verstanden werden, welche hinsichtlich ihrer optischen Eigenschaften, beispielsweise durch Beaufschlagung mit einer Spannung und/oder Strom, schaltbar ist. Die Abbildungslinse könnte vorzugsweise ein Formgedächtnis-Material umfassen. Bevorzugt ist eine Brennweite der Abbildungslinse schaltbar. Die Beleuchtungslinse weiste insbesondere einen ersten Schaltzustand auf, in welchem diese eine erste Brennweite umfasst, welche Licht gemäß dem ersten Transmissionsspektrums scharf auf die Bilderfassungssensorik abbildet. Ferner weist die Abbildungslinse insbesondere einen zweiten Schaltzustand auf, in welchem diese eine zweite Brennweite umfasst, welche von der ersten Brennweite verschieden ist, und welche Licht gemäß eines zweiten Transmissionsspektrums, welches von dem ersten Transmissionspektrum verschieden ist, scharf auf die Bilderfassungssensorik abbildet.

Weiterhin wird vorgeschlagen, dass die Filtereinheit zumindest einen weiteren Spektralfilter aufweist, welcher dazu ausgebildet ist, in einem weiteren ersten Betriebszustand Licht gemäß eines weiteren ersten spektralen Transmissionsbereichs zu transmittieren und in einem weiteren zweiten Betriebszustand dazu ausgebildet ist, Licht gemäß eines weiteren zweiten spektralen Transmissionsbereich zu transmittieren. Es kann vorteilhaft eine Bildgebung weiter verbessert werden, bei welchen verschiedenste Arten von Spektren zur Bildgebung genutzt werden können. Der weitere Spektralfilter ist bis auf seine optischen Eigenschaften insbesondere zumindest im Wesentlichen identisch zum Spektralfilter ausgebildet. Unter "zumindest im Wesentlichen identisch" soll bis auf Herstellung und/oder Montagetoleranzen identisch verstanden werden. Dabei unterscheiden sich der weitere erster Transmissionsbereich und der weitere zweite Transmissionsbereich in äquivalenter Weise voneinander, wie es auch der erste Transmissionsbereich des Spektralfilters und der zweite Transmissionsbereich des Spektralfilters tun und zwar insbesondere in Bezug auf Filterkanten, Bandbreiten und/oder Zentralwellenlänge des jeweiligen weiteren Transmissionsbereichs. Die optischen Eigenschaften des Spektralfilters sind dabei so gewählt, dass dieser insbesondere in dem ersten Betriebszustand zur Autofluoreszenzbildgebung und vorzugsweise in dem zweiten Betriebszustand zur Weißlichtbildgebung ausgebildet ist. Die optischen Eigenschaften des weiteren Spektralfilters sind dabei so gewählt, dass dieser insbesondere in dem weiteren ersten Betriebszustand zur Fluoreszenzbildgebung, vorzugsweise mittels Indocyaningrün, und vorzugsweise in dem zweiten Betriebszustand zu einer weiteren Fluoreszenzbildgebung, vorzugsweise mittels OTL38, ausgebildet ist. Insbesondere ist zumindest einer der weiteren Transmissionsbereiche des weiteren Spektralfilters von zumindest einem Transmissionsbereich der Transmissionsbereiche des weiteren Spektralfilters verschieden. Der erste Transmissionsbereich ist insbesondere im Vergleich zum weiteren ersten Transmissionsbereich in Richtung kleinerer Wellenlängen verschoben. Der erste Transmissionsbereich und der weitere erste Transmissionsbereich sind vorzugsweise frei von einer Überschneidung miteinander. Der zweite Transmissionsbereich ist insbesondere im Vergleich zum weiteren zweiter Transmissionsbereich in Richtung kleinerer Wellenlängen verschoben. Der zweite Transmissionsbereich und der weitere zweite Transmissionsbereich sind vorzugsweise frei von einer Überschneidung miteinander. Besonders bevorzugt sind die Transmissionsbereiche und die weiteren Transmissionsbereiche frei von einer Überschneidung miteinander.

Es wird ferner vorgeschlagen, dass die Bilderfassungseinheit zumindest eine weitere Bilderfassungssensorik umfasst, welche dem weiteren Spektralfilter zugeordnet und welcher in Blickrichtung der weiteren Bilderfassungssensorik betrachtet vor dieser angeordnet ist. Es kann vorteilhaft eine Effizienz einer Bildgebung weiter verbessert werden. Insbesondere ermöglicht die weitere Bilderfassungssensorik das Aufnehmen mit der Bilderfassungssensorik, sodass verschiedene Bildinformationen zugleich erfasst werden können. Die weitere Bilderfassungssensorik ist insbesondere zumindest im Wesentlichen identisch zur Bilderfassungssensorik ausgebildet. Insbesondere weist die Bilderfassungseinheit eine Anzahl von Bilderfassungssensoriken auf, welche der Anzahl von Spektralfiltern entspricht.

Insbesondere um eine gezielte Taktung der Bilderfassungseinheit zu gewährleisten, wird vorgeschlagen, dass die medizinische Bildgebungsvorrichtung wenigstens eine Steuereinheit umfasst, welche dazu ausgebildet ist, zumindest die Bilderfassungseinheit anzusteuern. Unter einer "Steuereinheit" soll insbesondere eine elektronische Einheit verstanden werden, welche zumindest einen Prozessor und vorzugsweise einen Speicher umfasst. Der Prozessor ist dazu ausgebildet, ein Betriebsprogramm zum Betrieb der Bilderfassungsvorrichtung auszuführen. Vorzugsweise ist das Betriebsprogramm auf dem Speicher hinterlegt.

Es wird vorgeschlagen, dass die Steuereinheit dazu ausgebildet ist, in Abhängigkeit von wenigstens einem Betriebszustand wenigstens eines Spektralfilters, insbesondere des Spektralfilters und/oder des weiteren Spektralfilters, der Filtereinheit zumindest eine Bilderfassungssensorik, insbesondere die Bilderfassungssensorik und/oder die weitere Bilderfassungssensorik, zu aktivieren oder zu deaktivieren. Es kann vorteilhaft eine Bilderfassung weiter verbessert werden. Insbesondere kann eine fehlerhafte Erfassung von nicht in dem Transmissionsbereich vorgesehenem transmittiertem Licht durch eine dauerhaft aktivierten Bilderfassungssensorik vermieden werden. Weiter vorteilhaft kann eine Flexibilität einer Bilderfassung verbessert werden. Die Steuereinheit steuert insbesondere die jeweilige Bilderfassungssensorik synchronisiert mit einem jeweiligen Betriebszustand eines jeweiligen Spektralfilters an. Vorzugsweise umfasst die Steuereinheit zumindest einen Betriebsmodus, welchem wenigstens ein Betriebszustand wenigstens eines Spektralfilters, insbesondere relativ zur wenigstens einer Bilderfassungssensorik, zugeordnet ist. Die Anzahl der Betriebsmodi entspricht dabei insbesondere der Anzahl aller möglichen Kombinationen der Betriebszustände der Spektralfilter. Die Anzahl der Betriebsmodi berechnet sich beispielsweise aus der Anzahl der Betriebszustände potenziert mit der Anzahl der Spektralfilter. Bevorzugt weist die Bildgebungsvorrichtung insgesamt zwei Spektralfilter mit jeweils zwei möglichen Betriebszuständen auf, woraus sich insbesondere eine Anzahl von vier verschiedenen Betriebsmodi ergibt. Es ergeben sich insbesondere Kombinationen der Betriebsmodi, wobei zeitgleiche Kombinationen von Betriebsmodi, in welchen sich der selbe Spektralfilter zeitgleich in verschiedenen Betriebzuständen befindet, ausgeschlossen sind. Im bevorzugten Fall können immer nur jeweils Betriebsmodi verschiedener Spektralfilter gleichzeitig durchgeführt werden. Vorzugsweise ist einem jeweiligen Betriebsmodus eine Aktivierung oder Deaktivierung der dem Spektralfilter zugeordneten Bilderfassungssensorik zugeordnet. Die Steuereinheit ist dazu ausgebildet, die Betriebsmodi untereinander abzuwechselnd und/oder zumindest teilweise gleichzeitig zu betreiben. Im bevorzugten Fall befindet sich in einem ersten Betriebsmodus der Spektralfilter in dem ersten Betriebszustand,, in einem zweiten Betriebsmodus der Spektralfilter in dem zweiten Betriebszustand,, in dem weiteren ersten Betriebsmodus der weitere Spektralfilte,r in dem weiteren ersten Betriebszustand und im weiteren zweiten Betriebsmodus der weitere Spektralfilter in dem weiteren zweiten Betriebszustand. Die Betriebsmodi sind insbesondere zur Bereitstellung verschiedener Darstellungsmethoden, wie beispielsweise Weißlichtdarstellung, Autofluoreszenzdarstellung und/oder Fluoreszendarstellung vorgesehen. Die Steuereinheit steuert dazu einen jeweiligen Aktor zur Überführung der Stellungen und/oder einer jeweiligen Bilderfassungssensorik entsprechend einer Taktung von zumindest 15 Hz, vorzugsweise zumindest 30 Hz und besonders bevorzugt zumindest 60 Hz an. Beispielsweise könnte die Steuereinheit gleichzeitig zwischen dem ersten Betriebsmodus und dem zweiten Betriebsmodus und/oder dem weiteren ersten und weiteren zweiten Betriebsmodus alternieren. Die Steuereinheit ist insbesondere dazu ausgebildet, anhand der von der Bilderfassungssensorik sensierten Bilderfassung eine Bildinformation bereitzustellen. Vorzugsweise separiert die Steuereinheit mittels einer selben Bilderfassungssensorik aufgenommene Bildinformationen zweier alternierender Betriebsmodi in zwei separate Bildinformationen. Beispielsweise könnte die Steuereinheit Bildinformationen verschiedener Betriebsmodi aber gleicher Bilderfassungssensorik zu einem Film mit einer entsprechenden Bildrate erstellen, welche zumindest 15 Hz, vorzugsweise zumindest 30 Hz und besonders bevorzugt zumindest 60 Hz entspricht. Alternativ oder zusätzlich könnte die Steuereinheit Bildinformationen zweier oder mehrerer Betriebsmodi gemeinsam, gleichzeitig und/oder überlagert ausgeben, und zwar insbesondere als Film mit einer entsprechenden Bildrate von zumindest 15 Hz, vorzugsweise zumindest 30 Hz und besonders bevorzugt 60 Hz.

Es wird zudem vorgeschlagen, dass die Steuereinheit dazu ausgebildet ist, in Abhängigkeit von wenigstens einem Betriebszustand,, insbesondere dem Betriebszustand und/oder dem weiteren Betriebszustand, wenigstens eines Spektralfilters, insbesondere des Spektralfilters und/oder des weiteren Spektralfilters, der Filtereinheit zumindest ein Beleuchtungslicht gemäß wenigstens eines Beleuchtungsspektrums der Beleuchtungseinheit zu aktivieren oder zu deaktivieren. Es kann vorteilhaft eine Bilderfassung weiter verbessert werden. Insbesondere kann eine fehlerhafte Erfassung von nicht für einen jeweiligen Transmissionsbereich vorgesehenem Beleuchtungslicht durch eine dauerhaft aktivierte Beleuchtung vermieden werden. Weiter vorteilhaft kann eine Flexibilität einer Bilderfassung verbessert werden. Die Steuereinheit steuert insbesondere das jeweilige Beleuchtungslicht bzw. das Beleuchtungslicht bereitstellende Beleuchtungselement der Beleuchtungseinheit synchronisiert mit einem jeweiligen Betriebszustand eines jeweiligen Spektralfilters an, wobei diese vorzugsweise zusätzlich von der Steuereinheit mit einer jeweiligen Bilderfassungssensorik synchronisiert werden. Die Steuereinheit kann das Beleuchtungslicht gemäß einem jeweiligen Betriebsmodus aktivieren und/oder deaktivieren, wobei eine Aktivierung oder Deaktivierung von verschiedenen Beleuchtungsspektren verschiedenen Betriebsmodi zugeordnet ist.

Weiterhin wird vorgeschlagen, dass die Steuereinheit dazu ausgebildet ist, in Abhängigkeit von wenigstens einem Betriebszustand wenigstens eines Spektralfilters einen Schaltzustand der schaltbaren Abbildungslinse zu aktivieren oder zu deaktivieren. Es kann vorteilhaft eine Bilderfassung weiter verbessert werden. Insbesondere kann eine fehlerhafte Erfassung von nicht für einen jeweiligen Transmissionsbereich vorgesehenen Brennweite durch eine gezielte Aktivierung der verschiedenen Schaltzustände der schaltbaren Abbildungslinse vermieden werden. Die Steuereinheit steuert insbesondere einen Schaltzustand der Abbildungslinse synchronisiert mit einem jeweiligen Betriebszustand eines jeweiligen Spektralfilters an, wobei dieser vorzugsweise von der Steuereinheit wiederum mit einer jeweiligen Bilderfassungssensorik und/oder Beleuchtungseinheit synchronisiert werden. Die Steuereinheit kann die Abbildungslinse gemäß eines Schaltzustands der Abbildungslinse verschiedenen Betriebsmodi zugeordnet ist.

Es wird vorgeschlagen, dass die medizinische Bildgebungsvorrichtung zumindest teilweise ein medizinisches Bildgebungsgerät, insbesondere ein endoskopisches Bildgebungsgerät, ausbildet. Hierdurch kann insbesondere ein medizinisches Bildgebungsgerät mit einer verbesserten Effizienz bereitgestellt werden.

In einem weiteren Aspekt der Erfindung wird ein Verfahren zum Betrieb einer medizinischen Bildgebungsvorrichtung, insbesondere einer endoskopischen Bildgebungsvorrichtung, beansprucht, bei welchem wenigstens ein Spektralfilter einer optischen Filtereinheit einer Bilderfassungssensorik zugeordnet und in Blickrichtung der Bilderfassungssensorik betrachtet vor dieser angeordnet ist.

Dabei wird vorgeschlagen, dass in wenigstens einem Verfahrensschritt der Spektralfilter in wenigstens einem ersten Betriebszustand, insbesondere relativ zur Bilderfassungssensorik, Licht gemäß eines ersten spektralen Transmissionsbereichs zur Bilderfassungssensorik transmittiert und in einem weiteren Verfahrensschritt der Spektralfilter in wenigstens einem zweiten Betriebszustand, insbesondere relativ zu Bilderfassungssensorik, Licht gemäß eines zweiten spektralen Transmissionsbereichs, welcher von dem ersten spektralen Transmissionsbereich zumindest teilweise verschieden ist, zur Bilderfassungssensorik transmittiert.

Hierdurch kann insbesondere ein Verfahren zur medizinischen Bilderfassungsvorrichtung mit einer verbesserten Effizienz bereitgestellt werden.

Die erfindungsgemäße Vorrichtung, sowie das erfindungsgemäße Verfahren sollen/soll hierbei nicht auf die oben beschriebene Anwendung und Ausführungsform beschränkt sein. Insbesondere kann die erfindungsgemäße Vorrichtung und/oder das erfindungsgemäße Verfahren zu einer Erfüllung einer hierin beschriebenen Funktionsweise eine von einer hierin genannten Anzahl von einzelnen Elementen, Bauteilen und Einheiten sowie Verfahrensschritten abweichende Anzahl aufweisen. Zudem sollen bei den in dieser Offenbarung angegebenen Wertebereichen auch innerhalb der genannten Grenzen liegende Werte als offenbart und als beliebig einsetzbar gelten.

Es wird insbesondere darauf hingewiesen, dass alle in Bezug auf die Vorrichtung beschriebenen Merkmale und Eigenschaften, aber auch Verfahrensweisen, sinngemäß auf das erfindungsgemäße Verfahren übertragbar und im Sinne der Erfindung einsetzbar und als mitoffenbart gelten. Gleiches gilt auch in umgekehrter Richtung. Das bedeutet, dass auch in Bezug auf das Verfahren genannte, bauliche also vorrichtungsgemäße Merkmale im Rahmen der Vorrichtungsansprüche berücksichtigt, beansprucht und ebenfalls zur Offenbarung gezählt werden können.

Bezüglich optischer Eigenschaften, sei darauf hingewiesen, dass diese Schwankungen unterworfen sein können und zwar insbesondere mit Bezug auf eine Fluoreszenz, welche von externen Eigenschaften, wie beispielsweise einer Konzentration eines Farbstoffs, einem verwendeten Lösungsmittels für den Farbstoff, einer Intensität einer Anregung zur Fluoreszenz, einer Wellenlänge zur Anregung der Fluoreszenz oder dergleichen.

Falls von einem bestimmten Objekt mehr als ein Exemplar vorhanden ist, ist nur eines davon in den Figuren und in der Beschreibung mit einem Bezugszeichen versehen. Die Beschreibung dieses Exemplars kann entsprechend auf die anderen Exemplare von dem Objekt übertragen werden. Sind Objekte insbesondere mittels Zahlenwörtern, wie beispielsweise erstes, zweites, drittes Objekt etc. benannt, dienen diese der Benennung und/oder Zuordnung von Objekten. Demnach kann beispielsweise ein erstes Objekt und ein drittes Objekt, jedoch kein zweites Objekt umfasst sein. Allerdings könnten anhand von Zahlenwörtern zusätzlich auch eine Anzahl und/oder eine Reihenfolge von Objekten ableitbar sein.

### Zeichnungen

Weitere Vorteile ergeben sich aus der folgenden Zeichnungsbeschreibung. In den Zeichnungen ist ein Ausführungsbeispiel der Erfindung dargestellt. Die Zeichnungen, die Beschreibung und die Ansprüche enthalten zahlreiche Merkmale in Kombination. Der Fachmann wird die Merkmale zweckmäßigerweise auch einzeln betrachten und zu sinnvollen weiteren Kombinationen zusammenfassen.

Es zeigen:
- Fig. 1: eine schematische Darstellung einer medizinischen Bildgebungsvorrichtung in einer perspektivischen Ansicht,
- Fig. 2: eine schematische Darstellung eines Teils der medizinischen Bildgebungsvorrichtung mit einer Bilderfassungseinheit in einer Draufsicht,
- Fig. 3: ein schematisches Diagramm, in welchem optische Eigenschaften der medizinischen Bilderfassungsvorrichtung gemäß eines ersten Betriebszustands eines Spektralfilters relativ zu einer Bilderfassungssensorik der Bilderfassungseinheit dargestellt sind,
- Fig. 4: ein schematisches Diagramm, in welchem optische Eigenschaften der medizinischen Bilderfassungsvorrichtung gemäß eines zweiten Betriebszustands eines Spektralfilters relativ zu einer Bilderfassungssensorik der Bilderfassungseinheit dargestellt sind,
- Fig. 5: ein schematisches Diagramm, in welchem optische Eigenschaften der medizinischen Bilderfassungsvorrichtung gemäß einsr ersten Betriebszustands eines weiteren Spektralfilters relativ zu einer weiteren Bilderfassungssensorik der Bilderfassungseinheit dargestellt sind,
- Fig. 6: ein schematisches Diagramm, in welchem optische Eigenschaften der medizinischen Bilderfassungsvorrichtung gemäß eines zweiten Betriebszustands eines Spektralfilters relativ zu einer Bilderfassungssensorik der Bilderfassungseinheit dargestellt sind,
- Fig. 7: einen ersten Betriebsmodus der medizinischen Bildgebungsvorrichtung in einer Draufsicht,
- Fig. 8: einen zweiten Betriebsmodus der medizinischen Bildgebungsvorrichtung in einer Draufsicht,
- Fig. 9: einen weiteren ersten Betriebsmodus der medizinischen Bildgebungsvorrichtung in einer Draufsicht,
- Fig. 10: einen weiteren zweiten Betriebsmodus der medizinischen Bildgebungsvorrichtung in einer Draufsicht,
- Fig. 11: einen schematischen Ablaufplan eines beispielhaften Verfahrens zum Betrieb der medizinischen Bildgebungsvorrichtung und
- Fig. 12: eine schematische Darstellung einer alternativen medizinischen Bildgebungsvorrichtung in einer perspektivischen Ansicht.

### Beschreibung der Ausführungsbeispiele

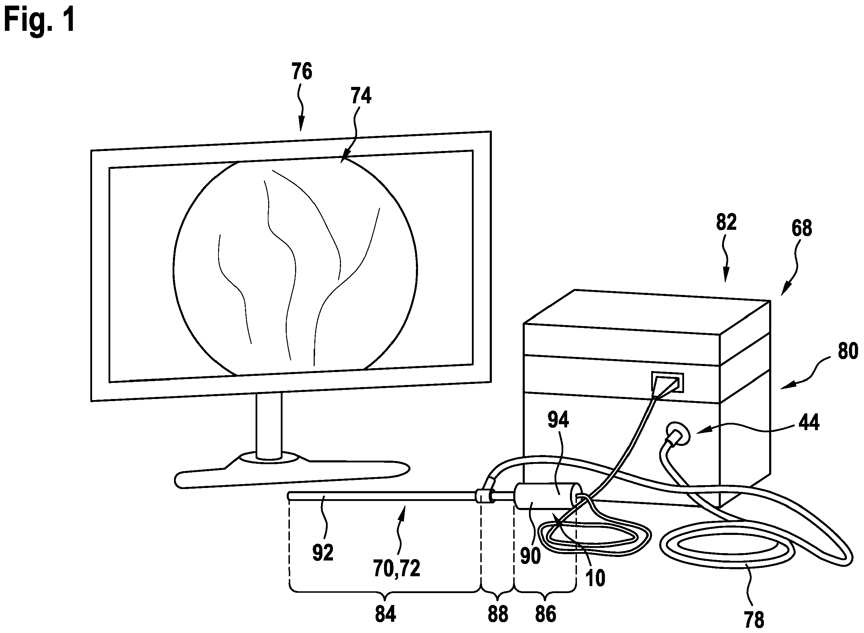

Fig. 1 zeigt eine schematische Darstellung einer medizinischen Bildgebungsvorrichtung in einer perspektivischen Ansicht. Bei der medizinischen Bildgebungsvorrichtung handelt es sich im vorliegenden Fall um eine endoskopische Bildgebungsvorrichtung. Alternativ könnte es sich bei der medizinischen Bildgebungsvorrichtung um eine exoskopische, eine mikroskopische oder eine makroskopische Bildgebungsvorrichtung handeln. Die medizinische Bildgebungsvorrichtung ist zu einer Untersuchung einer Kavität vorgesehen.

Die medizinische Bildgebungsvorrichtung umfasst zumindest ein medizinisches Bildgebungsgerät 70. Bei dem medizinischen Bildgebungsgerät 70 handelt es sich im vorliegenden Fall um ein Endoskop 72. Alternativ könnte es sich bei dem medizinischen Bildgebungsgerät 70 um ein Exoskop, ein Mikroskop und/oder ein Makroskop handeln.

Das medizinische Bildgebungsgerät 70 weist einen distalen Abschnitt 84auf. Der distale Abschnitt 84 ist dazu ausgebildet, in einem Betriebszustand in eine Kavität eingeführt zu werden. Der distale Abschnitt 84 ist in dem Betriebszustand einem Patienten zugewandt. Der distale Abschnitt 84 ist in dem Betriebszustand einem Bediener abgewandt. Ferner weist das medizinische Bildgebungsgerät 70 einen proximalen Abschnitt 86 auf. Der proximale Abschnitt 86 ist in dem Betriebszustand außerhalb einer Kavität angeordnet. Der proximale Abschnitt 86 ist in dem Betriebszustand einem Patienten abgewandt. Der proximale Abschnitt 86 ist in dem Betriebszustand einem Bediener zugewandt.

Das medizinische Bildgebungsgerät 70 weist einen Zwischenabschnitt 88 auf. Der Zwischenabschnitt 88 ist zwischen dem distalen Abschnitt 84 und dem proximalen Abschnitt 86 angeordnet.

Die medizinische Bildgebungsvorrichtung weist eine Verbindungseinheit 90 auf. Die Verbindungseinheit 90 ist Teil des medizinischen Bildgebungsgeräts 70. Die Verbindungseinheit 90 ist im Bereich des Zwischenabschnitts 88 angeordnet. Die Verbindungseinheit 90 ist zu einer Verbindung des proximalen Abschnitts 86 und des distalen Abschnitts 84 miteinander ausgebildet. Im vorliegenden Fall ist die Verbindungseinheit 90 zu einer form- und/oder kraftschlüssigen Verbindung des proximalen Abschnitts 86 und des distalen Abschnitts 84 miteinander ausgebildet, wie beispielsweise mittels einer Bajonettverbindung.

Die medizinische Bildgebungsvorrichtung weist zumindest einen Schaft 92 auf. Der Schaft 92 ist Teil des medizinischen Bildgebungsgeräts 70. Der Schaft 92 bildet zumindest teilweise den distalen Abschnitt 84 des endoskopischen Bildgebungsgeräts 70 aus.

Ferner weist die endoskopische Bildgebungsvorrichtung wenigstens eine Handhabe 94 auf. Die Handhabe 94 ist Teil des medizinischen Bildgebungsgeräts 70. Die Handhabe 94 bildet zumindest teilweise den proximalen Abschnitt 86 des medizinischen Bildgebungsgeräts 70 aus.

Die endoskopische Bildgebungsvorrichtung weist wenigstens eine Bilderfassungseinheit 10 auf. Die Bilderfassungseinheit 10 ist Teil des medizinischen Bilderfassungsgeräts 70. Die Bilderfassungseinheit 10 ist im Bereich des proximalen Abschnitts 86 angeordnet. Die Bilderfassungseinheit 10 ist in die Handhabe 94 integriert. Alternativ oder zusätzlich kann die Bilderfassungseinheit 10 zumindest teilweise im Bereich des distalen Abschnitts 84 angeordnet und zwar insbesondere in den Schaft 92 integriert sein.

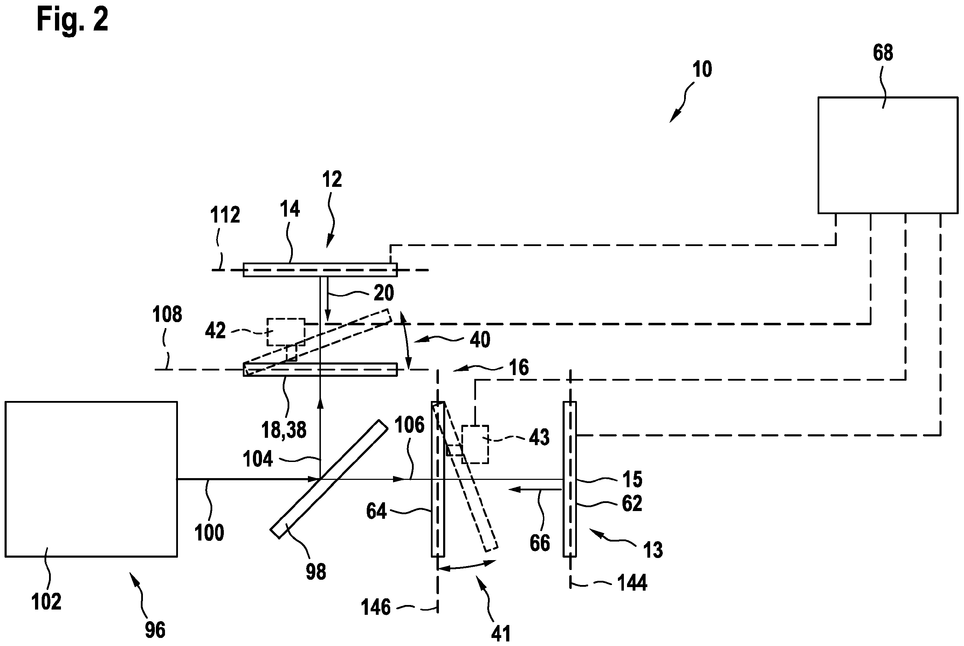

Fig. 2 zeigt eine schematische Darstellung eines Teils der endoskopischen Bildgebungsvorrichtung mit der Bilderfassungseinheit 10 in einer Draufsicht.

Zu einer Einkopplung von Licht umfasst die Bilderfassungseinheit 10 zumindest eine Bilderfassungsoptikeinheit 96. Die Bilderfassungsoptikeinheit 96 umfasst wenigstens eine Optik 102, wie beispielsweise ein Objektiv, ein Okular, eine Linse, ein Prisma, einen Lichtwellenleiter oder dergleichen. Die Bilderfassungsoptikeinheit 96 definiert einen Eingangsstrahlengang 100.

Die Bilderfassungsoptikeinheit 96 weist wenigstens einen Strahlteiler 98 auf. Im vorliegenden Fall ist der Strahlteiler 98 als ein Interferrenzstrahlteiler ausgebildet. In Einfallsrichtung des Eingangsstrahlengangs 100 betrachtet ist der Strahlteiler 98 vor der Optik 102 angeordnet. Der Strahlteiler 98 teilt den Eingangsstrahlengang 100 in zumindest zwei Strahlengänge 104, 106 und zwar einen Strahlengang 104 und einen weiteren Strahlengang 106 auf. Der Strahlteiler 98 teilt den Eingangsstrahlengang 100 zu im Wesentlichen gleichen Teilen in die zwei Strahlengänge 104, 106 auf. Alternativ könnte der Strahlteiler 98 den Eingangsstrahlengang 100 auch zu unterschiedlichen Teilen aufteilen.

Die Bilderfassungseinheit 10 weist wenigstens eine Bilderfassungssensorik 12 auf. Die Bilderfassungssensorik 12 ist dazu ausgebildet, Bildinformationen mit einer Bildrate von wenigstens 15 Bildern pro Sekunde aufzunehmen. Im vorliegenden Fall ist die Bilderfassungssensorik 12 sogar dazu ausgebildet, zumindest 30 Bilder pro Sekunde aufzunehmen. Die Bilderfassungssensorik 12 umfasst wenigstens einen Bildsensor 14. Im vorliegenden Fall ist der Bildsensor als ein CMOS-Sensor ausgebildet. Die Bilderfassungssensorik kann mehrere Bilderfassungssensoren aufweisen. Alternativ oder zusätzlich kann wenigstens ein Bildsensor 14 der Bilderfassungssensorik 12 als ein CCD-Sensor ausgebildet sein.

Die endoskopische Bildgebungsvorrichtung umfasst wenigstens eine optische Filtereinheit 16. Die optische Filtereinheit 16 ist zumindest teilweise zwischen der Bilderfassungsoptikeinheit 98 und der Bilderfassungssensorik 12 angeordnet. Die optische Filtereinheit 16 umfasst wenigstens einen Spektralfilter 18. Der Spektralfilter 18 ist zwischen der Optik 102 und der Bilderfassungssensorik 12 angeordnet. Der Spektralfilter 18 ist zwischen dem Strahlteiler 98 und der Bilderfassungssensorik 12 angeordnet. Der Spektralfilter 18 ist der Bilderfassungssensorik 12 zugeordnet. Der Spektralfilter 18 ist in einer Blickrichtung 20 der Bilderfassungssensorik 12 betrachtet vor dieser angeordnet. Der Spektralfilter 18 ist als ein Interferenzfilter 38 ausgebildet. Ferner handelt es sich bei dem Spektralfilter 18 um einen Bandfilter. Im vorliegenden Fall ist der Spektralfilter 18 von dem Strahlteiler 98 separat ausgebildet. Es wäre jedoch denkbar, dass der Strahlteiler 98 den Spektralfilter 18 ausbilden könnte, wodurch vorteilhaft Bauteile und/oder ein Bauraum reduziert werden können/kann, da insbesondere auch auf einen weiteren Spektralfilter verzichtet werden könnte.

Der Spektralfilter 18 weist wenigstens zwei Betriebszustände auf. Im vorliegenden Fall sind die Betriebszustände Stellungen des Spektralfilters 18 relativ zur Bilderfassungssensorik 12. Der Spektralfilter 18 weist einen ersten Betriebszustand auf. Der erste Betriebszustand ist definiert als eine erste Stellung des Spektralfilters 18 relativ zur Bilderfassungssensorik 12. Ferner weist der Spektralfilter 18 einen zweiten Betriebszustand auf. Der zweite Betriebszustand des Spektralfilters 18 ist definiert als eine zweite Stellung des Spektralfilters 18 relativ zur Bilderfassungssensorik 12. Der Spektralfilter 18 ist von dem ersten Betriebszustand in den zweiten Betriebszustand überführbar. Der Spektralfilter 18 ist von dem zweiten Betriebszustand in den ersten Betriebszustand überführbar. Ferner sind weitere Betriebszustände, insbesondere Stellungen des Spektralfilters 18 relativ zur Bilderfassungssensorik 12 denkbar, welche jeweils verschiedene Transmissionsbereiche aufweisen könnten. Beispielsweise könnte der Spektralfilter 18 relativ zur Bilderfassungssensorik 12 auch kontinuierlich verstellbar sein. Alternativ wäre es auch denkbar, dass um eine Stellung des Spektralfilters 18 relativ zur Bilderfassungssensorik 12 zu variieren anstelle des Spektralfilters 18 die Bilderfassungssensorik 12 von einer ersten Stellung in eine zweite Stellung und umgekehrt überführbar ist.

Im vorliegenden Fall ist der Spektralfilter 18 zur Überführung von dem ersten Betriebszustand in den zweiten Betriebszustand verkippbar. Ferner ist der Spektralfilter 18 von dem zweiten Betriebszustand in den ersten Betriebszustand rückkippbar. Der Spektralfilter 18 weist eine Haupterstreckungsebene 108 auf. Im vorliegenden Fall ist der Spektralfilter 18 um eine Schwenkachse 110 schwenkbar, welche zumindest im Wesentlichen parallel zu der Haupterstreckungsebene 108 ist. Im vorliegenden Fall ist die Schwenkachse 110 von einer Seitenkante des Spektralfilters 18 definiert. Alternativ ist es denkbar, dass der Spektralfilter 18 drehbar/verkippbar sein kann und zwar insbesondere um eine Gier-, Hoch- und/oder Vertikalachse des Spektralfilters 18.

Der erste Betriebszustand und der zweiten Betriebszustand sind definiert durch zueinander verschiedene Stellungen des Spektralfilters 18 relativ zur Bilderfassungssensorik 12. Der erste Betriebszustand und der zweite Betriebszustand unterscheiden sich durch eine jeweilige Orientierung einer Stellung des Spektralfilters 18 relativ zur Bilderfassungssensorik 12. Die Bilderfassungssensorik 12 weist eine Haupterstreckungsebene 112 auf. In dem ersten Betriebszustand des Spektralfilters 18 ist die Haupterstreckungsebene 108 des Spektralfilters 18 zumindest im Wesentlichen senkrecht zur optischen Achse. Im vorliegenden Fall ist in dem ersten Betriebszustand des Spektralfilters die Haupterstreckungsebene 108 des Spektralfilters 18 zumindest im Wesentlichen senkrecht zu einer Haupterstreckungsebene 112 der Bilderfassungssensorik 12. In dem zweiten Betriebszustand des Spektralfilters 18 ist die Haupterstreckungsebene 108 des Spektralfilters 18 winklig zur optischen Achse. Ferner ist in dem zweiten Betriebszustand des Spektralfilters 18 die Haupterstreckungsebene 108 des Spektralfilters 18 winklig zur Haupterstreckungsebene der Bilderfassungssensorik 12. In dem zweiten Betriebszustand beträgt ein Winkel zwischen der Haupterstreckungsebene 108 des Spektralfilters 18 und der optischen Achse der Bilderfassungssensorik 12 zumindest 92°. Der Winkel beträgt ferner höchstens 135°. Im vorliegenden Fall beträgt der Winkel im Wesentlichen 115°. Ferner beträgt in dem zweiten Betriebszustand ein Winkel zwischen der Haupterstreckungsebene 108 des Spektralfilters 18 und der Haupterstreckungsebene 112 der Bilderfassungssensorik 12 zumindest 2°. Der Winkel beträgt ferner höchstens 45°. Im vorliegenden Fall beträgt der Winkel im Wesentlichen 25°.

Der Spektralfilter ist von dem ersten Betriebszustand in den zweiten Betriebszustand überführbar. Dazu weist die Filtereinheit 16 wenigstens ein Lager 40 auf. Das Lager 40 ist dazu ausgebildet, den Spektralfilter 18 von dem ersten Betriebszustand in den zweiten Betriebszustand und/oder umgekehrt überführbar zu lagern. Das Lager 40 ist im vorliegenden Fall als ein als Schwenklager ausgebildet. Ferner könnte das Lager 40 auch als ein Kipp- und/oder Drehlager ausgebildet sein. Alternativ oder zusätzlich könnte das Lager 40 zu einer verstellbaren Lagerung der Bilderfassungssensorik 12 dienen.

Die Filtereinheit 16 weist wenigstens einen Aktor 42 auf. Der Aktor 42 ist dazu ausgebildet den Spektralfilter 18 von dem ersten Betriebszustand in den zweiten Betriebszustand und/oder umgekehrt zu überführen. Der Aktor 42 ist dazu ausgebildet, mindestens 15 mal pro Sekunde den Spektralfilter 18 von dem ersten Betriebszustand in den zweiten Betriebszustand des Spektralfilters 18 zu überführen. Der Aktor 42 ist ferner dazu ausgebildet, mindestens 15 mal pro Sekunde den Spektralfilter 18 von dem zweiten Betriebszustand in den ersten Betriebszustand des Spektralfilters 18 zu überführen. Demnach finden insgesamt mindestens 30 mal Wechsel der Betriebszustände des Spektralfilters 18 pro Sekunde statt. Der Aktor 42 ist beispielsweise als ein Schrittmotor ausgebildet. Schritte des Aktors 42 entsprechen dabei mit Winkelsegmenten des Winkels zwischen dem ersten Betriebszustand und dem zweiten Betriebszustand des Spektralfilters 18 relativ zur Bilderfassungssensorik 12.

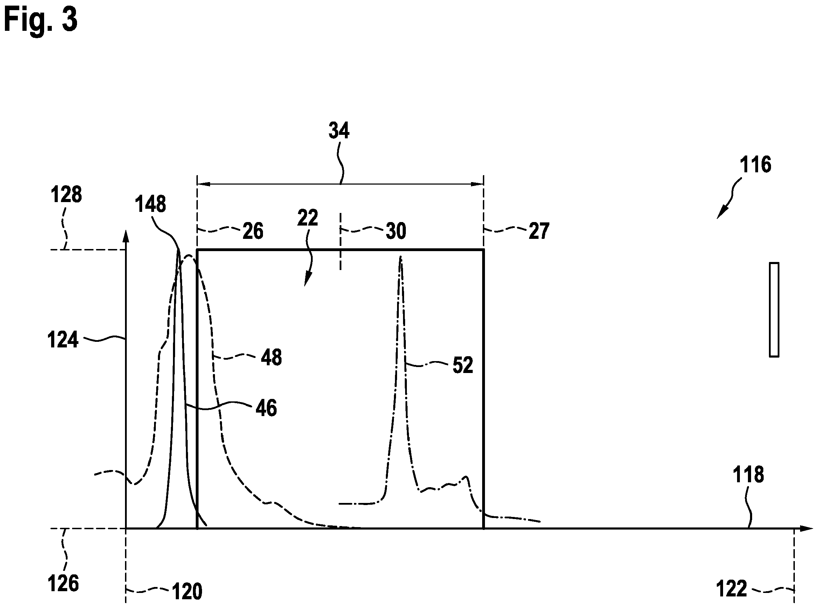

Fig. 3 zeigt ein schematisches Diagramm 116, in welchem optische Eigenschaften der medizinischen Bilderfassungsvorrichtung gemäß des ersten Betriebszustands des Spektralfilters 18, insbesondere relativ zur Bilderfassungssensorik 12 dargestellt sind. Das Diagramm 116 weist eine Abszissenachse 118 auf. Auf der Abszissenachse 118 ist eine Wellenlänge aufgetragen. Die Wellenlänge ist linear auf der Abszissenachse 118 aufgetragen. Die Wellenlänge ist auf der Abszissenachse 118 in der Einheit nm aufgetragen. Ein dargestellter Bereich der Abszissenachse 118 erstreckt sich von einem Minimalwert 120 bis zu einem Maximalwert 122. Der Minimalwert 120 beträgt 350 nm Der Maximalwert 122 beträgt 1050 nm. Das Diagramm 116 weist eine Ordinatenachse 124 auf. Auf der Ordinatenachse 124 ist eine Intensität aufgetragen. Die Intensität ist linear auf der Ordinatenachse aufgetragen. Die Intensität ist auf der Ordinatenachse 124 normiert aufgetragen. Dazu sind in dem Diagramm 116dargestellte Intensitätsverläufe auf ihr entsprechendes Intensitätsmaximum normiert. Die Intensität ist auf der Ordinatenachse 124 in willkürlichen Einheiten a.u. (arbitrary units) angegeben. Ein dargestellter Bereich der Ordinatenachse 124 erstreckt sich von einem Minimalwert 126 bis zu einem Maximalwert 128. Der Minimalwert 126 beträgt 0. Der Maximalwert 128 beträgt 1.

In dem ersten Betriebszustand weist der Spektralfilter 18 einen ersten Transmissionsbereich 22 auf. Der Transmissionsbereich ist gemäß einem Intensitätsverlauf im Diagramm 126 der Fig. 3 dargestellt. In dem ersten Betriebszustand ist der Spektralfilter 18 dazu ausgebildet, Licht gemäß des ersten spektralen Transmissionsbereichs 22 zur Bilderfassungssensorik 12 zu transmittieren. Der erste spektrale Transmissionsbereich 22 weist eine erste Filterkante 26 auf. Die erste Filterkante 26 liegt im Wesentlichen bei 430 nm. Bei der ersten Filterkante 26 handelt es sich um eine steigende Filterkante. Der erste spektrale Transmissionsbereich 22 weist eine erste Zentralwellenlänge 30 auf. Die Zentralwellenlänge 30 liegt im Wesentlichen bei 575 nm. Ferner weist der erste Transmissionsbereich 22 eine weitere erste Filterkante 27 auf. Die weitere erste Filterkante 27 liegt im Wesentlichen bei 725 nm. Bei der weiteren ersten Filterkante 27 handelt es sich um eine fallende Filterkante. Der erste spektrale Transmissionsbereich 22 weist eine erste Bandbreite 34 auf. Die erste Bandbreite 34 beträgt im Wesentlichen 295 nm.

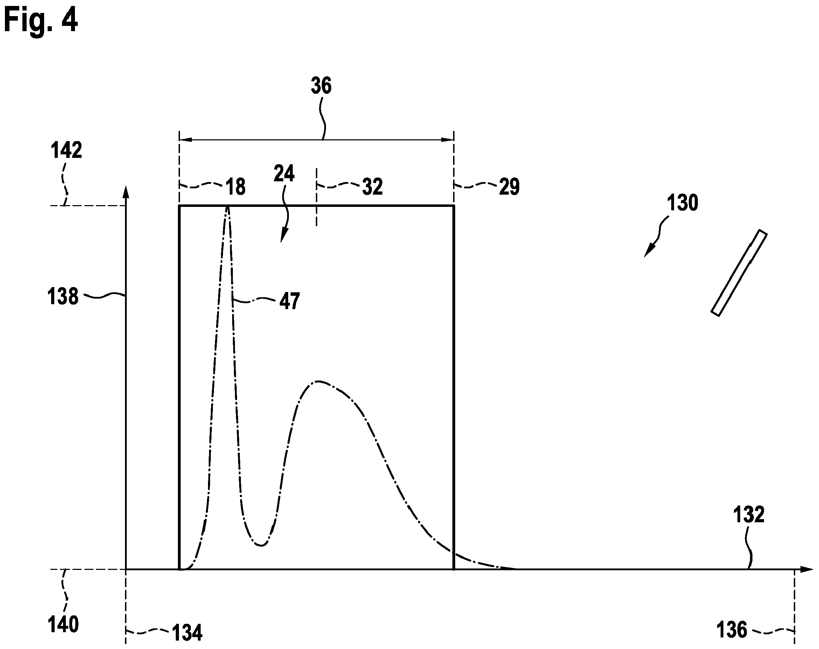

Fig. 4 zeigt ein schematisches Diagramm 130, in welchem optische Eigenschaften der medizinischen Bilderfassungsvorrichtung gemäß des zweiten Betriebszustands des Spektralfilters 18, insbesondere relativ zur Bilderfassungssensorik 12 dargestellt sind. Das Diagramm 130 weist eine Abszissenachse 132 auf. Auf der Abszissenachse 132 ist eine Wellenlänge aufgetragen. Die Wellenlänge ist linear auf der Abszissenachse 132 aufgetragen. Die Wellenlänge ist auf der Abszissenachse 132 in der Einheit nm aufgetragen. Ein dargestellter Bereich der Abszissenachse 132 erstreckt sich von einem Minimalwert 134 bis zu einem Maximalwert 136. Der Minimalwert 134 beträgt 350 nm. Der Maximalwert 136 beträgt 1050 nm. Das Diagramm 116 weist eine Ordinatenachse 138 auf. Auf der Ordinatenachse 138 ist eine Intensität aufgetragen. Die Intensität ist linear auf der Ordinatenachse 138 aufgetragen. Die Intensität ist auf der Ordinatenachse 138 normiert aufgetragen. Dazu sind in dem Diagramm dargestellte Intensitätsverläufe auf ihr entsprechendes Intensitätsmaximum normiert. Die Intensität ist auf der Ordinatenachse 138 in willkürlichen Einheiten a.u. (arbitrary units) angegeben. Ein dargestellter Bereich der Ordinatenachse 138 erstreckt sich von einem Minimalwert 140 bis zu einem Maximalwert 142. Der Minimalwert 140 beträgt 0. Der Maximalwert 142 beträgt 1.

In dem zweiten Betriebszustand ist der Spektralfilter 18 dazu ausgebildet Licht gemäß einem zweiten spektralen Transmissionsbereich 24 Licht zur Bilderfassungssensorik 12 zu transmittieren. Der Transmissionsbereich ist gemäß einem Intensitätsverlauf im Diagramm 130 der Fig. 4 dargestellt. Der zweite spektrale Transmissionsbereich 24 weist eine zweite Filterkante 28 auf. Bei der zweiten Filterkante 28 handelt es sich um eine steigende Filterkante. Die zweite Filterkante 28 liegt im Wesentlichen bei 400 nm. Der zweite spektrale Transmissionsbereich 24 weist eine zweite Zentralwellenlänge 32 auf. Die zweite Zentralwellenlänge 32 liegt im Wesentlichen bei 542 nm. Die weitere zweite Filterkante 29 liegt im Wesentlichen bei 670 nm. Der zweite spektrale Transmissionsbereich 24 weist eine weitere zweite Filterkante 29 auf. Bei der weiteren zweiten Filterkante 29 handelt es sich um eine fallende Filterkante. Der zweite spektrale Transmissionsbereich 24 weist eine zweite Bandbreite 36 auf. Die zweite Bandbreite beträgt im Wesentlichen 283 nm.

Der zweite spektrale Transmissionsbereich 24 ist von dem ersten spektralen Transmissionsbereich 22 zumindest teilweise verschieden. Die zweite Filterkante 28 ist relativ zur ersten Filterkante 26 spektral verschoben. Die zweite Filterkante 28 ist um im Wesentlichen 30 nm zur ersten Filterkante 26 verschoben. Die zweite Zentralwellenlänge 32 ist relativ zur ersten Zentralwellenlänge 30 spektral verschoben. Die zweite Zentralwellenlänge 32 ist zur ersten Zentralwellenlänge 30 um im Wesentlichen 33 nm verschoben. Die zweite Bandbreite 36 ist zumindest teilweise von der ersten Bandbreite 34 verschieden. Die zweite Bandbreite 36 ist kleiner als die erste Bandbreite 34. Die zweite Bandbreite 36 ist um im Wesentlichen 5 % kleiner als die erste Bandbreite 34. Die zweite Bandbreite 36 ist im Wesentlichen um 12 nm kleiner als die erste Bandbreite 34. Die zweite Bandbreite 36 entspricht zumindest zu einem Großteil der ersten Bandbreite 34. Die zweite Bandbreite 36 überlappt zu wenigstens 90 % mit der ersten Bandbreite 34.

Die Beleuchtungseinheit 44 ist dazu ausgebildet, wenigstens ein erstes Beleuchtungslicht gemäß eines ersten Beleuchtungsspektrums 46 bereitzustellen. Das erste Beleuchtungsspektrum 46 ist gemäß einem Intensitätsverlauf im Diagramm 116 der Fig. 3 dargestellt. Dazu weist die Beleuchtungseinheit 44 wenigstens ein erstes Beleuchtungselement auf, wie beispielsweise eine LED, eine Laserdiode, eine Laser oder dergleichen, welche Licht gemäß dem Beleuchtungsspektrum 46 emittiert. Das erste Beleuchtungsspektrum 46 weist einen gaußschen Intensitätsverlauf auf. Das erste Beleuchtungsspektrum 46 weist einen Peak 148 auf. Der Peak 148 liegt im vorliegenden Fall im Wesentlichen bei 410 nm. Das erste Beleuchtungsspektrum 46 liegt zumindest zu einem Großteil außerhalb des ersten spektralen Transmissionsbereichs 22 in dem ersten Betriebszustand des Spektralfilters 18 relativ zur Bilderfassungssensorik 12. Demnach ist in dem ersten Betriebszustand des Spektralfilters 18, insbesondere relativ zur Bilderfassungssensorik 12 Licht gemäß dem ersten Beleuchtungsspektrum zumindest zu einem Großteil herausgefiltert. Im Gegensatz dazu, liegt das Beleuchtungsspektrum 46 zumindest zu einem Großteil innerhalb des zweiten spektralen Transmissionsbereichs 24 in dem zweitem Betriebszustand des Spektralfilters 18, insbesondere relativ zur Bilderfassungssensorik 12 (vgl. Fig. 4).

Ein erstes Absorptionsspektrum 48 eines spontan Licht emittierenden Materials eines zu erfassenden Objekts ist gemäß einem Intensitätsverlauf im Diagramm 116 der Fig. 3 dargestellt. Das erste Beleuchtungsspektrum 46 entspricht zumindest zu einem Großteil dem ersten Absorptionsspektrum 48 des spontan Licht emittierenden Materials des zu erfassenden Objekts. Im vorliegenden Fall handelt es sich bei dem spontan Licht emittierenden Material des erfassenden Objekts um Gewebe, welches eine Autofluoreszenz zeigt. Das Gewebe umfasst dabei Porphyrin und zwar insbesondere Protoporphyrin. Das erste Absorptionsspektrum 48 liegt zumindest zu einem Großteil außerhalb des ersten spektralen Transmissionsbereichs 22 in dem ersten Betriebszustand des Spektralfilters 18, insbesondere relativ zur Bilderfassungssensorik 12. Insbesondere im Gegensatz dazu, liegt das erste Absorptionsspektrum 48 zumindest zu einem Großteil innerhalb des zweiten spektralen Transmissionsbereichs 24 in dem zweiten Betriebszustand des Spektralfilters 18, insbesondere relativ zur Bilderfassungssensorik 12 (vgl. Fig. 4).

Ein Emissionsspektrum 52 des mittels der Beleuchtungseinheit 44 beleuchteten spontan Licht emittierenden Materials ist gemäß einem Intensitätsverlauf im Diagramm 116 in einem Bereich von im Wesentlichen 620 nm bis im Wesentlichen 750 nmn in der Fig. 3 dargestellt. Das Emissionsspektrum 52 des mittels der Beleuchtungseinheit 44 beleuchteten spontan Licht emittierenden Materials liegt zumindest zu einem Großteil im ersten spektralen Transmissionsbereich 22. Demnach kann von dem spontan Licht emittierenden Material des erfassenden Objekts emittiertes Licht des Emissionsspektrums 52 zumindest zu einem Großteil den ersten Transmissionsbereich 22 des Spektralfilters 18 passieren. Insbesondere im Gegensatz dazu, liegt das Emissionsspektrum 52 zumindest zu einem Großteil außerhalb des zweiten spektralen Transmissionsbereichs 24 in dem zweiten Betriebszustand des Spektralfilters 18, insbesondere relativ zur Bilderfassungssensorik 12 (vgl. Fig. 4).

Die Beleuchtungseinheit 44 ist dazu ausgebildet, wenigstens ein zweites Beleuchtungslicht eines zweiten Beleuchtungsspektrums 47 bereitzustellen. Das zweite Beleuchtungsspektrum 47 ist gemäß einem Intensitätsverlauf im Diagramm 130 der Fig. 4 dargestellt. Dazu weist die Beleuchtungseinheit 44 wenigstens ein zweites Beleuchtungselement auf. Bei dem zweiten Beleuchtungselement handelt es sich um eine Weißlichtquelle, wie beispielsweise eine LED-Röhrenlampe, eine Leuchtstofflampe oder dergleichen. Das zweite Beleuchtungsspektrum 47 liegt zumindest zu einem Großteil innerhalb des zweiten spektralen Transmissionsbereichs 24. Bei dem zweiten Beleuchtungsspektrum 47 handelt es sich um ein Weißlichtspektrum. Demnach lässt der zweite spektrale Transmissionsberiech 24 Licht gemäß dem zweiten Beleuchtungsspektrum 47 zumindest zu einem Großteil passieren. Insbesondere im Gegensatz dazu, liegt das zweite Beleuchtungsspektrum 47 zumindest teilweise außerhalb des ersten Transmissionsbereichs 22.

Wie in Fig. 2 dargestellt, weist die Bilderfassungseinheit 10 zumindest eine weitere Bilderfassungssensorik 62 auf. Die weitere Bilderfassungssensorik 62 ist zumindest im Wesentlichen identisch zur Bilderfassungssensorik 12 ausgebildet. Die weitere Bilderfassungssensorik 62 weist wenigstens einen weiteren Bildsensor 15 auf. Die weitere Bilderfassungssensorik 62 ist in dem weiteren Strahlengang 106 angeordnet. Die weitere Bilderfassungssensorik 62 ist versetzt zu Bilderfassungssensorik 12 angeordnet. Die weitere Bilderfassungssensorik 62 weist eine Haupterstreckungsebene 144 auf. Die Haupterstreckungsebene 144 der weiteren Bilderfassungssensorik 62 ist im vorliegenden Fall zumindest im Wesentlichen senkrecht zur Haupterstreckungsebene 112 der Bilderfassungssensorik 12.

Die Filtereinheit 16 weist zumindest einen weiteren Spektralfilter 64 auf. Der weitere Spektralfilter 64 ist im vorliegenden Fall von dem Spektralfilter 18 verschieden ausgebildet. Der weitere Spektralfilter 64 unterscheidet sich von dem Spektralfilter 18 durch seine optischen Eigenschaften. Für den Fall, dass der Strahlteiler 98 den Spektralfilter 18 ausbildet, würde dieser ebenfalls den weiteren Spektralfilter 64 ausbilden.

Der weitere Spektralfilter 64 ist im weiteren Strahlengang 106 angeordnet. Die weitere Bilderfassungssensorik 62 ist dem weiteren Spektralfilter 64 zugeordnet. Der weitere Spektralfilter 64 ist in einer Blickrichtung 66 der weiteren Bilderfassungssensorik 62 betrachtet vor diesem angeordnet. Eine Haupterstreckungsebene 146 des weiteren Spektralfilters 64 ist zumindest im Wesentlichen parallel zur Haupterstreckung 108 des weiteren Spektralfilters 64 in zumindest einem Betriebszustand des Spektralfilters 12.

Der weitere Spektralfilter 64 weist wenigstens zwei Betriebszustände, insbesondere relativ zur weiteren Bilderfassungssensorik 62 auf und zwar einen weiteren ersten Betriebszustand und einen weiteren zweiten Betriebszustand. Der weitere erste Betriebszustand und der weitere zweite Betriebszustand des weiteren Spektralfilters 64, insbesondere relativ zur weiteren Bilderfassungssensorik 62, sind äquivalent zu dem ersten Betriebszustand und dem zweiten Betriebszustand des Spektralfilters 18 zur Bilderfassungssensorik 12.

Zur Lagerung des weiteren Spektralfilters 64 weist die Filtereinheit 16 wenigstens ein weiteres Lager 41 auf. Das weitere Lager 41 ist zumindest im Wesentlichen identisch zum Lager 40 ausgebildet. Zu Überführung des weiteren Spektralfilters 64 von dem weiteren ersten Betriebszustand in den weitere zweiten Betriebszustand und umgekehrt weist die Filtereinheit 16 wenigstens einen weiteren Aktor 43 auf. Der weitere Aktor 43 ist zumindest im Wesentlichen identisch zum Aktor 42 ausgebildet.

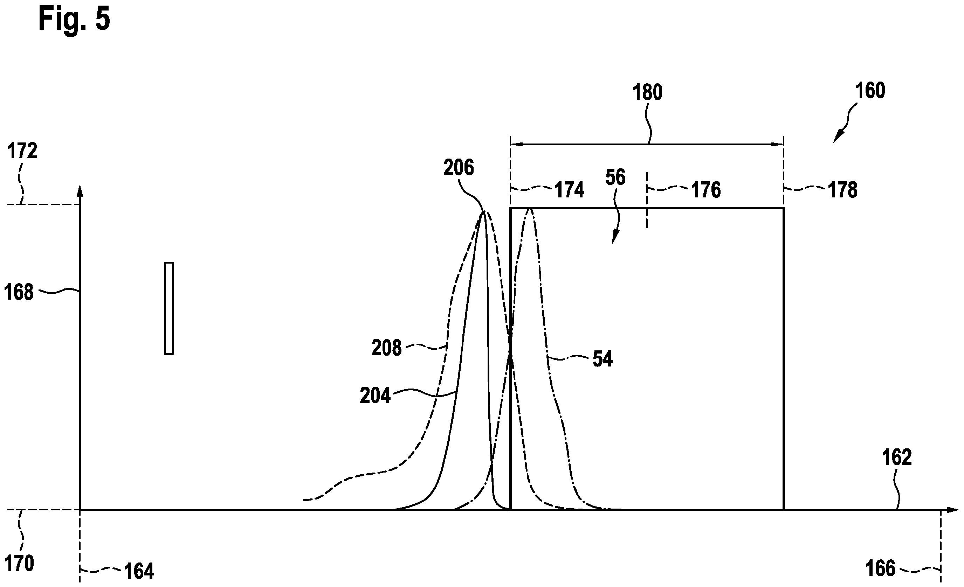

Fig. 5 zeigt ein schematisches Diagramm 160, in welchem optische Eigenschaften der medizinischen Bilderfassungsvorrichtung gemäß des ersten Betriebszustands des weiteren Spektralfilters 64, insbesondere relativ zur weiteren Bilderfassungssensorik 62 dargestellt sind. Das Diagramm 160 weist eine Abszissenachse 162 auf. Auf der Abszissenachse 162 ist eine Wellenlänge aufgetragen. Die Wellenlänge ist linear auf der Abszissenachse 162 aufgetragen. Die Wellenlänge ist auf der Abszissenachse 162 in der Einheit nm aufgetragen. Ein dargestellter Bereich der Abszissenachse 162 erstreckt sich von einem Minimalwert 164 bis zu einem Maximalwert 166. Der Minimalwert 164 beträgt 350 nm Der Maximalwert 166 beträgt 1250 nm. Das Diagramm 160 weist eine Ordinatenachse 168 auf. Auf der Ordinatenachse 168 ist eine Intensität aufgetragen. Die Intensität ist linear auf der Ordinatenachse 168 aufgetragen. Die Intensität ist auf der Ordinatenachse 168 normiert aufgetragen. Dazu sind in dem Diagramm 160 dargestellte Intensitätsverläufe auf ihr entsprechendes Intensitätsmaximum normiert. Die Intensität ist auf der Ordinatenachse 168 in willkürlichen Einheiten a.u. (arbitrary units) angegeben. Ein dargestellter Bereich der Ordinatenachse 168 erstreckt sich von einem Minimalwert 170 bis zu einem Maximalwert 172. Der Minimalwert 170 beträgt 0. Der Maximalwert 172 beträgt 1.

In dem ersten Betriebszustand weist der weitere Spektralfilter 64 einen weiteren ersten spektralen Transmissionsbereich 56 auf. Der weitere erste spektrale Transmissionsbereich 56 ist gemäß einem Intensitätsverlauf im Diagramm 160 der Fig. 5 dargestellt. In dem weiteren ersten Betriebszustand ist der weitere Spektralfilter 64 dazu ausgebildet, Licht gemäß des weiteren ersten spektralen Transmissionsbereichs 56 zur weiteren Bilderfassungssensorik 62 zu transmittieren. Der weitere erste spektrale Transmissionsbereich 56 weist eine weitere erste Filterkante 174 auf. Die weitere erste Filterkante 174 liegt im Wesentlichen bei 800 nm. Bei der weiteren ersten Filterkante 174 handelt es sich um eine steigende Filterkante. Der weitere erste spektrale Transmissionsbereich 56 weist eine weitere erste Zentralwellenlänge 176 auf. Die weitere erste Zentralwellenlänge 176 liegt im Wesentlichen bei 950 nm. Ferner weist der weitere erste Transmissionsbereich 56 eine weitere weitere erste Filterkante 178 auf. Die weitere weitere erste Filterkante 178 liegt im Wesentlichen bei 1100 nm. Bei der weiteren weiteren ersten Filterkante 178 handelt es sich um eine fallende Filterkante. Der weitere erste spektrale Transmissionsbereich 56 weist eine weitere erste Bandbreite 180 auf. Die weitere erste Bandbreite 180 beträgt im Wesentlichen 300 nm.

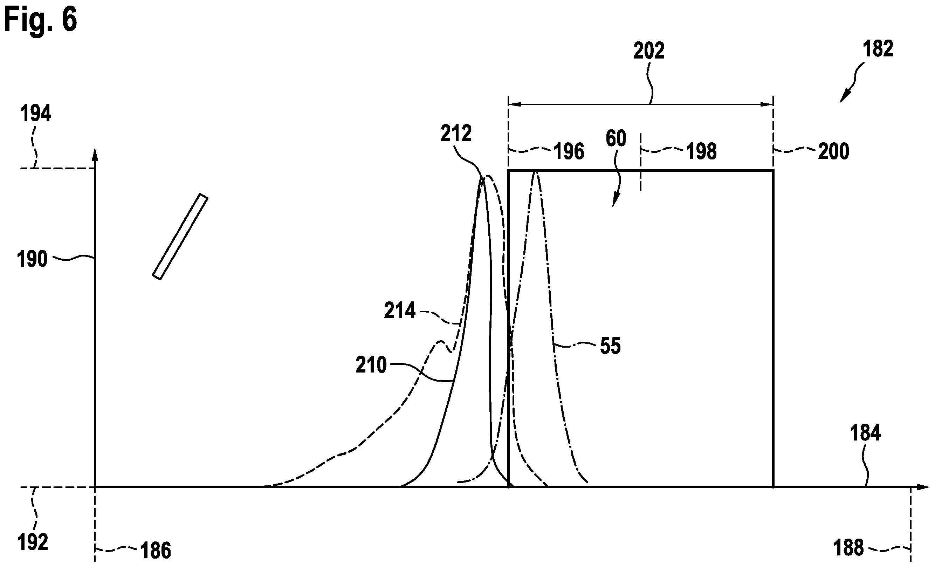

Fig. 6 zeigt ein schematisches Diagramm 182, in welchem optische Eigenschaften der medizinischen Bilderfassungsvorrichtung gemäß des weiteren zweiten Betriebszustands des weiteren Spektralfilters 64, insbesondere relativ zur weiteren Bilderfassungssensorik 62 dargestellt sind. Das Diagramm 182 weist eine Abszissenachse 184 auf. Auf der Abszissenachse 184 ist eine Wellenlänge aufgetragen. Die Wellenlänge ist linear auf der Abszissenachse 184 aufgetragen. Die Wellenlänge ist auf der Abszissenachse 184 in der Einheit nm aufgetragen. Ein dargestellter Bereich der Abszissenachse 184 erstreckt sich von einem Minimalwert 186 bis zu einem Maximalwert 188. Der Minimalwert 186 beträgt 350 nm. Der Maximalwert 188 beträgt 1250 nm. Das Diagramm 182 weist eine Ordinatenachse 190 auf. Auf der Ordinatenachse 190 ist eine Intensität aufgetragen. Die Intensität ist linear auf der Ordinatenachse 190 aufgetragen. Die Intensität ist auf der Ordinatenachse 190 normiert aufgetragen. Dazu sind in dem Diagramm 182 dargestellte Intensitätsverläufe auf ihr entsprechendes Intensitätsmaximum normiert. Die Intensität ist auf der Ordinatenachse 190 in willkürlichen Einheiten a.u. (arbitrary units) angegeben. Ein dargestellter Bereich der Ordinatenachse 190 erstreckt sich von einem Minimalwert 192 bis zu einem Maximalwert 194. Der Minimalwert 192 beträgt 0. Der Maximalwert 194 beträgt 1.

In dem weiteren zweiten Betriebszustand ist der weitere Spektralfilter 64 dazu ausgebildet, Licht gemäß eines weiteren zweiten spektralen Transmissionsbereichs 60 Licht zur weiteren Bilderfassungssensorik 62 zu transmittieren. Der weitere zweite spektrale Transmissionsbereich 60 ist gemäß einem Intensitätsverlauf im Diagramm 182 der Fig. 6 dargestellt. Der weitere zweite spektrale Transmissionsbereich 60 weist eine weitere zweite Filterkante 196 auf. Bei der weiteren zweiten Filterkante 196 handelt es sich um eine steigende Filterkante. Die weitere zweite Filterkante 196 liegt im Wesentlichen bei 770 nm. Der weitere zweite spektrale Transmissionsbereich 60 weist eine weitere zweite Zentralwellenlänge 198 auf. Die weitere zweite Zentralwellenlänge 198 liegt im Wesentlichen bei 900 nm. Der weitere zweite spektrale Transmissionsbereich 60 weist eine weitere weitere zweite Filterkante 200 auf. Bei der weiteren weiteren zweiten Filterkante 200 handelt es sich um eine fallende Filterkante. Die weitere weitere zweite Filterkante 200 liegt im Wesentlichen bei 1030 nm. Der weitere zweite spektrale Transmissionsbereich 60 weist eine weitere zweite Bandbreite 202 auf. Die weitere zweite Bandbreite 202 beträgt im Wesentlichen 283 nm.

Der weitere zweite spektrale Transmissionsbereich 60 ist von dem weiteren ersten spektralen Transmissionsbereich 56 zumindest teilweise verschieden. Die weitere zweite Filterkante 200 ist relativ zur weiteren ersten Filterkante 174 spektral verschoben. Die weitere zweite Filterkante 200 ist um im Wesentlichen 30 nm zur weiteren ersten Filterkante 174 verschoben. Die weitere zweite Zentralwellenlänge 198 ist relativ zur weiteren ersten Zentralwellenlänge 176 spektral verschoben. Die weitere zweite Zentralwellenlänge 198 ist zur weiteren ersten Zentralwellenlänge 176 um im Wesentlichen 30 nm verschoben. Die weitere zweite Bandbreite 202 ist zumindest teilweise von der weiteren ersten Bandbreite 180 verschieden. Die weitere zweite Bandbreite 202 ist kleiner als die weitere erste Bandbreite 180. Die weitere zweite Bandbreite 202 ist um im Wesentlichen 5 % kleiner als die weitere erste Bandbreite 180. Die weitere zweite Bandbreite 202 ist im Wesentlichen um 30 nm kleiner als die weitere erste Bandbreite 180. Die weitere zweite Bandbreite 202 entspricht zumindest zu einem Großteil der weiteren ersten Bandbreite 180. Die weitere zweite Bandbreite 202 überlappt zu wenigstens 90 % mit der weiteren ersten Bandbreite 180.

Die Beleuchtungseinheit 44 ist dazu ausgebildet, wenigstens ein weiteres erstes Beleuchtungslicht gemäß eines weiteren ersten Beleuchtungsspektrums 204 bereitzustellen. Das weitere erste Beleuchtungsspektrums 204 ist gemäß einem Intensitätsverlauf im Diagramm 160 der Fig. 5 dargestellt. Dazu weist die Beleuchtungseinheit 44 wenigstens ein weiteres erstes Beleuchtungselement auf, wie beispielsweise eine LED, eine Laserdiode, eine Laser oder dergleichen, welche Licht gemäß dem weiteren ersten Beleuchtungsspektrums 204 emittiert. Das weitere erste Beleuchtungsspektrums 204 weist einen gaußschen Intensitätsverlauf auf. Das weitere erste Beleuchtungsspektrum 204 weist einen Peak 206 auf. Der Peak 206 liegt im vorliegenden Fall im Wesentlichen bei 770 nm. Das weitere erste Beleuchtungsspektrums 204 liegt zumindest teilweise und vorzugsweise zumindest zu einem Großteil außerhalb des ersten spektralen Transmissionsbereichs 56 in dem weiteren ersten Betriebszustand des weiteren Spektralfilters 64, insbesondere relativ zur weiteren Bilderfassungssensorik 62. Demnach ist in dem weiteren ersten Betriebszustand des weiteren Spektralfilters 64, insbesondere relativ zur weiteren Bilderfassungssensorik 62, Licht gemäß dem weiteren ersten Beleuchtungsspektrum 204 zumindest zu einem Großteil herausgefiltert. Insbesondere im Gegensatz dazu, liegt das weitere erste Beleuchtungsspektrum 204 zumindest zu einem Großteil innerhalb des weiteren zweiten spektralen Transmissionsbereichs 60 in dem zweiten Betriebszustand des weiteren Spektralfilters 18, insbesondere relativ zur weiteren Bilderfassungssensorik 62 (vgl. Fig. 6).

Ein weiteres erstes Absorptionsspektrum 208 eines weiteren ersten spontan Licht emittierenden Materials eines zu erfassenden Objekts ist gemäß einem Intensitätsverlauf im Diagramm 160 der Fig. 5 dargestellt. Das weitere erste Beleuchtungsspektrum 204 entspricht zumindest zu einem Großteil dem weiteren ersten Absorptionsspektrum 208 des weiteren ersten spontan Licht emittierenden Materials des zu erfassenden Objekts. Im vorliegenden Fall handelt es sich bei dem weiteren ersten spontan Licht emittierenden Material um einen Fluoreszenzfarbstoff. Der Fluoreszenzfarbsoff ist Indocyaningrün. Das weitere erste Absorptionsspektrum 208 liegt zumindest zu einem Großteil außerhalb des weiteren ersten spektralen Transmissionsbereichs 56 in dem weiteren ersten Betriebszustand des weiteren Spektralfilters 64, insbesondere relativ zur weiteren Bilderfassungssensorik 13. Insbesondere im Gegensatz dazu, liegt das weitere erste Absorptionsspektrum 208 zumindest zu einem Großteil innerhalb des weiteren zweiten spektralen Transmissionsbereichs 60 in dem weiteren zweiten Betriebszustand des weiteren Spektralfilters 64, insbesondere relativ zur weiteren Bilderfassungssensorik 62 (vgl. Fig. 5).

Ein weiteres erstes Emissionsspektrum 54 des mittels der Beleuchtungseinheit 44 beleuchteten weiteren ersten spontan Licht emittierenden Materials ist gemäß einem Intensitätsverlauf im Diagramm 130 in einem Bereich von 750 nm bis 900 nm in der Fig. 4 dargestellt. Das weitere erste Emissionsspektrum 54 des mittels der Beleuchtungseinheit 44 beleuchteten weiteren ersten spontan Licht emittierenden Materials liegt zumindest zu einem Großteil im weiteren ersten spektralen Transmissionsbereich 56. Demnach kann von dem weiteren ersten spontan Licht emittierenden Material emittiertes Licht des weiteren ersten Emissionsspektrums 54 zumindest zu einem Großteil den weiteren ersten Transmissionsbereich 56 des weiteren Spektralfilter 64 passieren.

Die Beleuchtungseinheit 44 ist dazu ausgebildet, wenigstens ein weiteres zweites Beleuchtungslicht gemäß eines weiteren zweiten Beleuchtungsspektrums 210 bereitzustellen. Das weitere zweite Beleuchtungsspektrum 210 ist gemäß einem Intensitätsverlauf im Diagramm 182 der Fig. 6 dargestellt. Dazu weist die Beleuchtungseinheit 44 wenigstens ein weiteres zweites Beleuchtungselement auf, wie beispielsweise eine LED, eine Laserdiode, ein Laser oder dergleichen, welche Licht gemäß dem weiteren zweites Beleuchtungsspektrums 210 emittiert. Das weitere zweite Beleuchtungsspektrums 210 weist einen gaußschen Intensitätsverlauf auf. Das weitere zweite Beleuchtungsspektrums 201 weist einen Peak 212 auf. Der Peak 212 liegt im vorliegenden Fall im Wesentlichen bei 745 nm. Das weitere zweite Beleuchtungsspektrums 210 liegt zumindest zu einem Großteil außerhalb des weiteren zweiten spektralen Transmissionsbereichs 60 in dem weiteren zweiten Betriebszustand des weiteren Spektralfilters 64, insbesondere relativ zur weiteren Bilderfassungssensorik 62. Demnach ist in dem weiteren zweiten Betriebszustand des weiteren Spektralfilters 64, insbesondere relativ zur weiteren Bilderfassungssensorik 62, Licht gemäß dem weiteren ersten Beleuchtungsspektrum 204 zumindest zu einem Großteil herausgefiltert.

Ein weiteres zweites Absorptionsspektrum 214 eines weiteren zweiten spontan Licht emittierenden Materials eines zu erfassenden Objekts ist gemäß einem Intensitätsverlauf im Diagramm 160 der Fig. 5 dargestellt. Das weitere zweite Beleuchtungsspektrum 210 entspricht zumindest zu einem Großteil dem weiteren zweiten Absorptionsspektrum 214 des weiteren zweiten spontan Licht emittierenden Materials. Im vorliegenden Fall handelt es sich bei dem weiteren zweiten spontan Licht emittierenden Material um einen Fluoreszenzfarbstoff. Der Fluoreszenzfarbsoff ist OTL38. Das weitere zweite Absorptionsspektrum 214 liegt zumindest zu einem Großteil außerhalb des weiteren zweiten spektralen Transmissionsbereichs 60 in dem zweiten Betriebszustand des weiteren Spektralfilters 64, insbesondere relativ zur weiteren Bilderfassungssensorik 62.

Ein weiteres zweites Emissionsspektrum 55 des mittels der Beleuchtungseinheit 44 beleuchteten weiteren zweiten spontan Licht emittierenden Materials ist gemäß einem Intensitätsverlauf im Diagramm 182 in einem Bereich von 800 nm bis 900 nm in der Fig. 6 dargestellt. Das weitere zweite Emissionsspektrum 55 des mittels der Beleuchtungseinheit 44 beleuchteten weiteren zweiten spontan Licht emittierenden Materials liegt zumindest zu einem Großteil im weiteren zweiten spektralen Transmissionsbereich 60. Demnach kann von dem weiteren zweiten spontan Licht emittierenden Material des erfassenden Objekts emittiertes Licht des weiteren zweiten Emissionsspektrums 55 zumindest zu einem Großteil den weiteren zweiten Transmissionsbereich 60 des weiteren Spektralfilter 64 passieren.

Fig. 1 zeigt ferner eine Steuereinheit 68 der medizinischen Bildgebungsvorrichtung. Die Steuereinheit 68 ist zur Steuerung weiterer Komponenten der medizinischen Bildgebungsvorrichtung ausgebildet. Im vorliegenden Fall bildet die Steuereinheit 68 ein separates Steuergerät 82 aus. Alternativ könnte die Steuereinheit 68 Teil des medizinischen Bildgebungsgeräts 70 sein und/oder in einem gemeinsamen Gehäuse mit der Beleuchtungseinheit 44 angeordnet sein. Die Steuereinheit 68 umfasst wenigstens eine Steuerelektronik (nicht dargestellt). Die Steuerelektronik umfasst zumindest einen Prozessor. Ferner umfasst die Steuereinheit 68 zumindest einen Speicher. Auf dem Speicher ist ein Betriebsprogramm hinterlegt. Der Prozessor ist zu einer Ausführung des Betriebsprogramms ausgebildet.

Die Steuereinheit 68 ist zu einer Steuerung der Beleuchtungseinheit 44 ausgebildet. Dazu ist die Steuereinheit 68 elektrisch und/oder elektronisch mit der Beleuchtungseinheit 44 verbunden. Die Steuereinheit 68 ist dazu ausgebildet abhängig von einem Betriebsmodus das erste Beleuchtungselement, das zweite Beleuchtungselement, das weitere erste und/oder weitere zweite Beleuchtungselement zu aktivieren und/oder zu deaktivieren, um Beleuchtungslicht eines jeweiligen Beleuchtungsspektrums bereit zu stellen.

Die Steuereinheit 68 ist zu einer Steuerung der Bilderfassungseinheit 10 ausgebildet. Die Steuereinheit 68 ist elektrisch und/oder elektronisch mit der Bilderfassungseinheit 10 verbunden. Die Steuereinheit 68 steuert die Aktoren an, um die Spektralfilter in die jeweilige erste und/oder zweite Betriebszustand, insbesondere relativ zu den jeweiligen Bilderfassungssensoren zu überführen. Die Steuereinheit 68 steuert die Bilderfassungssensorik 12 und/oder die weitere Bilderfassungssensorik 62 an. Insbesondere aktiviert und/oder deaktiviert die Steuereinheit 68 die Bilderfassungssensorik 12 und/oder die weitere Bilderfassungssensorik 62.

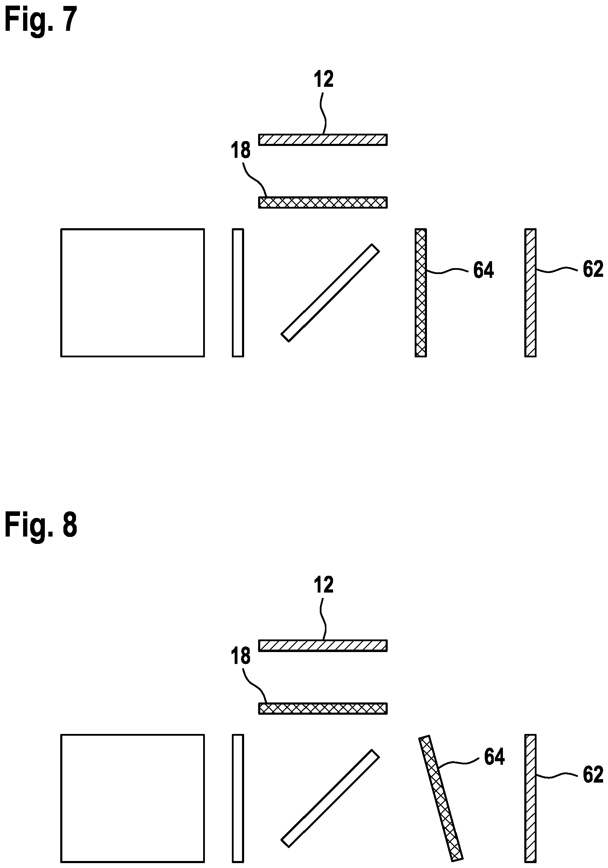

Fig. 7 bis 10 zeigen verschiedene mögliche Kombinationen der Betriebszustände der Spektralfilter 18, 64 relativ zueinander, sowie in verschiedenen Betriebsmodi. In Fig.7 befindet sich der Spektralfilter 18 in dem ersten Betriebszustand. Dies entspricht einem ersten Betriebsmodus. Der weitere Spektralfilter 64 befindet sich in dem weiteren ersten Betriebszustand. Dies entspricht einem weiteren ersten Betriebsmodus.

In Fig. 8 befindet sich der Spektralfilter 18 in dem zweiten Betriebszustand. Dies entspricht einem zweiten Betriebsmodus. Der weitere Spektralfilter 64 befindet sich in dem ersten Betriebszustand. Dies entspricht einem weiteren ersten Betriebsmodus.

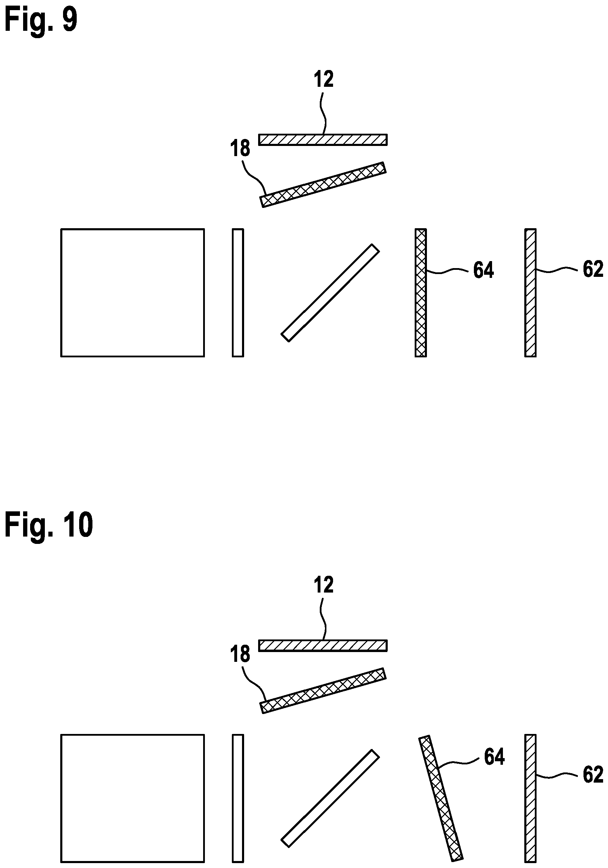

In Fig. 9 befindet sich der Spektralfilter 18 in dem ersten Betriebszustand. Dies entspricht dem ersten Betriebsmodus. Der weitere Spektralfilter 64 befindet sich in dem weiteren zweiten Betriebszustand Dies entspricht einem weiteren zweiten Betriebsmodus.

In Fig. 10 befindet sich der Spektralfilter 18 in der zweiten Stellung. Dies entspricht dem ersten Betriebszustand. Der weiteren Spektralfilter 64 befindet sich in dem weiteren zweiten Betriebszustand.

Die Steuereinheit 68 ist dazu ausgebildet, in Abhängigkeit von wenigstens einem Betriebszustand wenigstens eines der Spektralfilter 18, 64 der Filtereinheit 16 zumindest eine Bilderfassungssensorik 12 zu aktivieren oder zu deaktivieren. Die Steuereinheit 68 steuert dazu die Spektralfilter 18, 64 und die Bilderfassungssensorik 12 synchronisiert an. Ferner ist die Steuereinheit 68 dazu ausgebildet, in Abhängigkeit von einem Betriebszustand der Spektralfilter 18, 64 zumindest ein Beleuchtungslicht gemäß wenigstens eines Beleuchtungsspektrums der Beleuchtungseinheit 44 zu aktivieren oder zu deaktivieren. Die Steuereinheit 68 steuert dazu die Spektralfilter 18, 64 und die Beleuchtungseinheit synchronisiert an.

Die medizinische Bildgebungsvorrichtung weist ferner zumindest eine Anzeigeeinheit 74 auf. Die Anzeigeeinheit 74 ist zum Anzeigen von mit dem medizinischen Bildgebungsgerät 70 erfassten Bildinformationen ausgebildet. Im vorliegenden Fall bildet die Anzeigeeinheit 74 ein separates Anzeigegerät 76 aus. Alternativ könnte die Anzeigeeinheit 74 Teil des medizinischen Bildgebungsgeräts 70 sein. Die Steuereinheit 68 ist zu einer Steuerung der Anzeigeeinheit 74 ausgebildet. Dazu ist die Steuereinheit 68 elektrisch und/oder elektronisch mit der Anzeigeeinheit 74 verbunden. Die Steuereinheit 68 ist dazu ausgebildet, abhängig von einem Betriebsmodus mittels der Bilderfassungseinheit erfasste Bildinformation der Anzeigeeinheit 74 bereitzustellen.

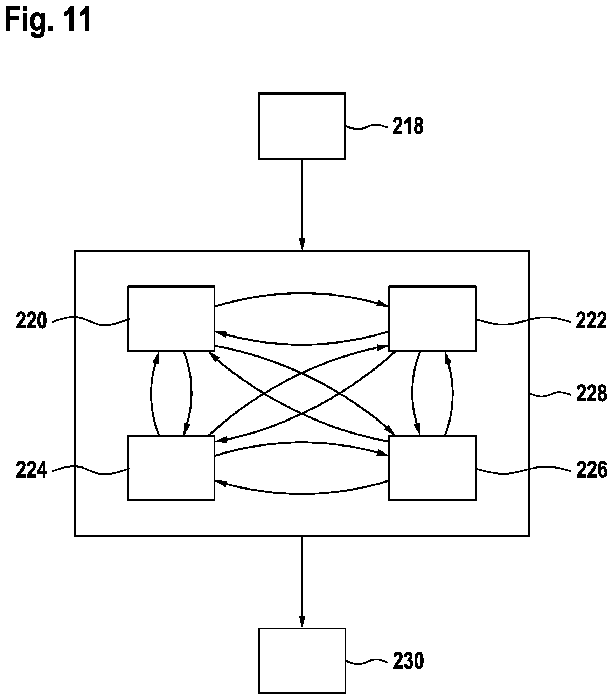

Fig. 11 zeigt einen schematischen Ablaufplan eines beispielhaften Verfahrens zum Betrieb der medizinischen Bildgebungsvorrichtung. Das Verfahren ist Teil eines Betriebsprogramms, welches in der Steuereinheit 68 hinterlegt und ausführbar ist.

Das Verfahren weist einen ersten Verfahrensschritt 218 auf. In dem Verfahrensschritt 218 wird eine Bildgebungsfunktion 228 gestartet.

Die Bildgebungsfunktion 228 umfasst zumindest einen weiteren Verfahrensteilschritt. In dem weiteren Verfahrensteilschritt 220 findet ein erster Betriebsmodus Anwendung. Im ersten Betriebsmodus steuert die Steuereinheit 68 das erste Beleuchtungselement an. Ferner steuert die Steuereinheit 68 den Aktor 42 an, sodass der Spektralfilter in dem ersten Betriebszustand überführt wird oder sich in dieser befindet. Ferner steuert die Steuereinheit 68 die Bilderfassungssensorik 12 an. Die Steuereinheit 68 aktiviert das erste Beleuchtungselement, sodass dieses Beleuchtungslicht entsprechend dessen ersten Beleuchtungsspektrum 46 bereitstellt. Da Beleuchtungslicht trifft auf ein zu untersuchendes Objekt. Das erste Beleuchtungsspektrum 46 wird zumindest teilweise von dem zu untersuchenden Objekt absorbiert. Das erste Beleuchtungsspektrum 46 regt das zu untersuchende Objekt zu Autofluoreszenz an. In dem ersten Betriebszustand entspricht der erste spektrale Transmissionsbereich 22 im Wesentlichen dem ersten Emissionsspektrum 52 der Autofluoreszenz. Ferner aktiviert die Steuereinheit 68 die Bilderfassungssensorik 12. Die Bilderfassungssensorik 12 sensiert Licht einer Bildinformation entsprechen dem ersten spektralen Transmissionsbereich 22.

Die Bildgebungsfunktion 228 umfasst zumindest einen weiteren Verfahrensteilschritt 222. In dem weiteren Verfahrenssteilschritt 222 findet ein zweiter Betriebsmodus Anwendung. Die Steuereinheit 68 steuert den Aktor 42 derart an, dass dieser den Spektralfilter 18 in dem zweite Betriebszustand überführt oder sich in dieser befindet. Ferner steuert die Steuereinheit 68 die Bilderfassungssensorik 12 an. Die Steuereinheit 68 aktiviert das zweite Beleuchtungselement, sodass dieses zweites Beleuchtungslicht entsprechend dessen zweiten Beleuchtungsspektrum 47 bereitstellt. Das zweite Beleuchtungslicht trifft auf ein zu untersuchendes Objekt. Das zweite Beleuchtungsspektrum 47 wird zumindest teilweise von dem zu untersuchenden Objekt reflektiert. In dem zweiten Betriebszustand des Spektralfilters 18 entspricht der zweite spektrale Transmissionsbereich 24 im Wesentlichen dem Reflexionsspektrum der des von dem zu untersuchenden Objekts reflektierten Beleuchtungslichts. Ferner aktiviert die Steuereinheit 68 die Bilderfassungssensorik 12. Die Bilderfassungssensorik 12 sensiert Licht einer Bildinformation entsprechen dem zweiten spektralen Transmissionsbereichs 24.

Das Verfahren weist einen weiteren Verfahrenssteilschritt 224 auf. In dem weiteren Verfahrenssteilschritt 224 findet ein weiterer erster Betriebsmodus Anwendung. Die Steuereinheit 68 steuert den weiteren Aktor 43 derart an, dass dieser den weiteren Spektralfilter 64 in den weiteren ersten Betriebszustand überführt oder sich dieser in dieser befindet. Ferner steuert die Steuereinheit 68 die weitere Bilderfassungssensorik 13 an. Die Steuereinheit 68 aktiviert das weitere erste Beleuchtungselement, sodass dieses weitere erste Beleuchtungslicht entsprechend dessen weiteren ersten Beleuchtungsspektrums bereitstellt. Das weitere erste Beleuchtungslicht trifft auf das zu untersuchendes Objekt. Das zu untersuchende Objekt umfasst ein weiteres erstes spontan Licht emittierendes Material. Im vorliegenden Fall ist das weitere erste spontan Licht emittierende Material Indocyaningrün. Das weitere erste Beleuchtungsspektrum 204 wird zumindest teilweise von dem zu untersuchenden Objekt absorbiert. Das weitere erste Beleuchtungsspektrum 204 entspricht zumindest zu einem Großteil einem weiteren ersten Absorptionsspektrum 208 des weiteren ersten spontan Licht emittierenden Materials. In dem weiteren ersten Betriebszustand entspricht der weitere erste spektrale Transmissionsbereich 56 im Wesentlichen einem weiteren ersten Emissionsspektrums 54 des weiteren ersten spontan Licht emittierenden Materials. Ferner aktiviert die Steuereinheit die weitere Bilderfassungssensorik. Die weitere Bilderfassungssensorik 13 sensiert Licht einer Bildinformation entsprechend dem weiteren ersten spektralen Transmissionsbereich 56.

Das Verfahren weist einen weiteren Verfahrensteilschrittschritt 226 auf. In dem weiteren Verfahrensteilschritt 226 findet ein weiterer zweiter Betriebsmodus Anwendung. Die Steuereinheit 68 steuert den weiteren Aktor 43 derart an, dass der weitere Spektralfilter 64 in den weiteren zweiten Betriebszustand überführt wird oder sich in dieser befindet. Ferner steuert die Steuereinheit 68 die weiteren Bilderfassungssensorik 13 an. Die Steuereinheit 68 aktiviert das weitere zweite Beleuchtungselement, sodass dieses weitere zweite Beleuchtungslicht entsprechend dessen weiteren zweiten Beleuchtungsspektrums 210 bereitstellt. Das weitere zweite Beleuchtungslicht trifft auf das zu untersuchende Objekt. Das zu untersuchende Objekt umfasst ein weiteres zweites spontan Licht emittierendes Material. Im vorliegenden Fall ist das weitere zweite spontan Licht emittierende Material OTL38. Das weitere zweite Beleuchtungsspektrum 210 wird zumindest teilweise von dem weiteren zweiten spontanen Licht emittierenden Material absorbiert. Das weitere zweite Beleuchtungsspektrum 210 entspricht zumindest zu einem Großteil einem weiteren zweiten Absorptionsspektrum 210 des weiteren zweiten spontan Licht emittierenden Materials. In dem weiteren zweiten Betriebszustand entspricht der weitere zweite spektrale Transmissionsbereich 60 im Wesentlichen einem weiteren zweite Emissionsspektrums 55 des weiteren zweiten spontan Licht emittierenden Materials. Ferner aktiviert die Steuereinheit 68 die weitere Bilderfassungssensorik 13. Die weitere Bilderfassungssensorik 13 sensiert Licht einer Bildinformation entsprechend dem weiteren zweiten spektralen Transmissionsbereich 60.

Zur Durchführung der Bildgebungsfunktion führt die Steuereinheit 68 die vorbenannten Verfahrensteilschritte nach Bedarf abwechselnd oder gleichzeitig aus, sodass entsprechende gleichzeitige Bereitstellung von verschiedenen Bildinformationen verschiedener Betriebsmodi und somit verschiedener spektraler Transmissionsbereiche und Beleuchtungsspektren erzielt werden. Die Bildinformationen werden der Anzeigeeinheit zur Verfügung gestellt. Auf der Anzeigeeinheit werden die zu Verfügung gestellten Bildinformationen dargestellt. Die Steuereinheit 68 stellt der Anzeigeeinheit je Betriebsmodus Bildinformationen als ein Film mit wenigstens einer Bildrate von 15 Bilder pro Sekunde zur Verfügung. Ferner kann die Steuereinheit 68 die Bildinformationen wenigstens zweier verschiedener Betriebsmodi kombinieren und als einen kombinierten Film mit wenigstens einer Bildrate von 30 Bildern pro Sekunde zur Verfügung stellen.

Das Verfahren umfasst einen weiteren Verfahrensschritt 230. In dem weiteren Verfahrensschritt 230 wird die Bildgebungsfunktion 228 beendet.

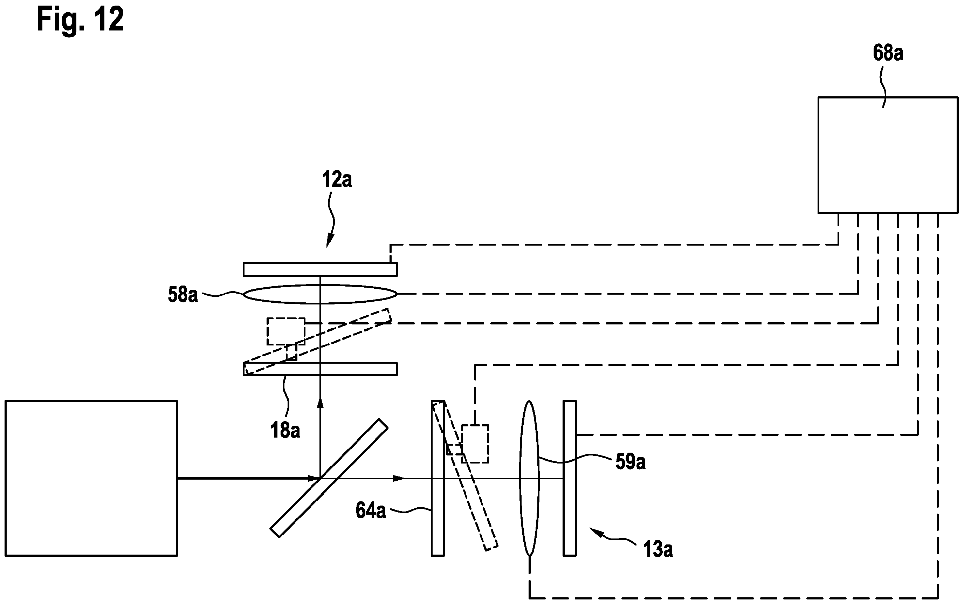

In Figur 12 ist ein weiteres Ausführungsbeispiel der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der anderen Ausführungsbeispiele, insbesondere der Figuren 1 bis 11 verwiesen wird. Zur Unterscheidung der Ausführungsbeispiele ist in Fig. 12 der den Bezugszeichen der Buchstabe a nachgestellt.

Fig. 12 zeigt ein weiteres Ausführungsbeispiel der Erfindung. Das vorliegende Ausführungsbeispiel unterscheidet sich von dem Vorhergehenden im Wesentlichen durch eine Ausgestaltung der Bilderfassungseinheit 10a. Im vorliegenden Fall weist die Filtereinheit 16a eine schaltbare Abbildungslinse 58a auf. Die schaltbare Abbildungslinse 58a ist zwischen dem Spektralfilter 18a und dem Bildsensor 14a angeordnet. Die schaltbare Abbildungslinse 58a ist dazu vorgesehen chromatische Abbildungsfehler, bei der Verwendung verschiedener Transmissionsspektren zu verringern oder zu vermeiden. Die schaltbare Abbildungslinse 58a ist dazu ausgebildet, abhängig von einem Betriebszustand des Spektralfilter 18a, ein von dem Spektralfilter 18a transmittiertes Transmissionsspektrum auf die Bilderfassungssensorik 12a abzubilden. Die schaltbare Abbildungslinse 58a ist hinsichtlich ihrer optischen Eigenschaften, beispielsweise durch Beaufschlagung mit einer Spannung und/oder einem Strom, schaltbar. Beispielsweise kann die schaltbare Abbildungslinse 58a ein Formgedächtnis-Material umfassen. Im vorliegenden Fall ist eine Brennweite der schaltbaren Abbildungslinse 58a schaltbar. Die schaltbare Abbildungslinse 58a weist insbesondere einen ersten Schaltzustand auf, in welchem diese einen ersten Brennwert umfasst, welche Licht gemäß dem ersten Transmissionsspektrums scharf auf die Bilderfassungssensorik 12a abbildet. Ferner weist die schaltbare Abbildungslinse 58a einen zweiten Schaltzustand auf, in welchem diese eine zweite Brennweite umfasst, welche von der ersten Brennweite verschieden ist, und welche Licht gemäß eines zweiten Transmissionsspektrums, welches von dem ersten Transmissionspektrum verschieden ist, scharf auf die Bilderfassungssensorik 12a abbildet. Die schaltbare Abbildungslinse 58a ist mit der Steuereinheit elektrisch und/oder elektronisch verbunden. Die schaltbare Abbildungslinse 58a ist mittels der Steuereinheit ansteuerbar bzw. schaltbar.

Ferner weist die Filtereinheit 16a wenigstens eine weitere schaltbare Abbildungslinse 59a auf. Die weitere schaltbare Abbildungslinse 59a ist zwischen dem weiteren Spektralfilter 18a und der weiteren Bilderfassungssensorik 62a angeordnet. Die weitere schaltbare Abbildungslinse 59a weist insbesondere einen weiteren ersten Schaltzustand auf, in welchem diese einen weiteren ersten Brennwert umfasst, welcher Licht gemäß dem weiteren ersten Transmissionsspektrums scharf auf die weitere Bilderfassungssensorik 62a abbildet. Ferner weist die weitere schaltbare Abbildungslinse 59a einen weiteren zweiten Schaltzustand auf, in welchem diese eine zweite Brennweite umfasst, welche von der weiteren ersten Brennweite verschieden ist, und welche Licht gemäß eines weiteren zweiten Transmissionsspektrums, welches von dem weiteren ersten Transmissionsspektrums verschieden ist, scharf auf die weitere Bilderfassungssensorik 62a abbildet. Die schaltbare Abbildungslinse 58a ist mit der Steuereinheit elektrisch und/oder elektronisch verbunden. Die schaltbare Abbildungslinse 58a ist mittels der Steuereinheit ansteuerbar bzw. schaltbar.

Um einen chromatischen Abbildungsfehler zu beheben, schaltet die Steuereinheit 68 in Abhängigkeit von wenigstens einem Betriebszustand der Spektralfilter 18, 64 einen Schaltzustand der schaltbaren Abbildungslinsen 58a, 59a zu aktivieren oder zu deaktivieren. Dies erfolgt synchronisiert mit der Ansteuerung der Beleuchtung, der Spektralfilter 18a. 64a und/oder der Bilderfassungssensoren 12a, 62a in jeweiligen Betriebsmodi, sodass dieser Vorgang Teil eines der Verfahrensschritte 220, 222, 224, 226 sein kann (vgl. Fig. 11).

| | | | |
|---|---|---|---|
| 10 | Bilderfassungseinheit | 43 | Weiterer Aktor |
| 12 | Bilderfassungssensorik | 44 | Beleuchtungseinheit |
| 13 | Weitere Bilderfassungssensorik | 46 | Erstes Beleuchtungsspektrum |
| 15 | Weiterer Bildsensor | 47 | Zweites Beleuchtungsspektrum |
| 14 | Bildsensor | 48 | Absorptionsspektrum |
| 16 | Filtereinheit | 52 | Emissionsspektrum |
| 18 | Spektralfilter | 54 | Weiteres erstes Emissionsspektrum |
| 20 | Blickrichtung | | |
| 22 | Erster spektraler Transmissionsbereich | 55 | Weiteres zweites Emissionsspektrum |
| 24 | Zweiter spektraler Transmissionsbereich | 56 | erster spektraler Transmissionsbereich |
| 26 | Erste Filterkante | 58 | Abbildungslinse |
| 27 | Weitere erste Filterkante | 59 | Weitere Abbildungslinse |
| 28 | Zweite Filterkante | 60 | Weiterer zweiter spektraler Transmissionsbereich |
| 29 | Weitere zweite Filterkante | 62 | Weitere Bilderfassungssensorik |
| 30 | Erste Zentralwellenlänge | | |
| 32 | Zweite Zentralwellenlänge | 64 | Weiterer Spektralfilter |
| 34 | Erste Bandbreite | 66 | Blickrichtung |
| 36 | Zweite Bandbreite | 68 | Steuereinheit |
| 38 | Interferenzfilter | 70 | Medizinisches Bildgebungsgerät |
| 40 | Lager | | |
| 41 | Lager | 72 | Endoskop |
| 42 | Aktor | 74 | Anzeigeeinheit |
| 76 | Anzeigegerät | 122 | Maximalwert der Abszissenachse |
| 78 | Lichtleiter | | |
| 80 | Beleuchtungsgerät | 124 | Ordinatenachse |
| 82 | Steuergerät | 126 | Minimalwert der Ordinatenachse |
| 84 | Distaler Abschnitt | 128 | Maximalwert der Ordinatenachse |
| 86 | Proximaler Abschnitt | | |
| 88 | Zwischenabschnitt | 130 | Diagramm |
| 90 | Verbindungseinheit | 132 | Abszissenachse |
| 92 | Schafft | 134 | Minimalwert der Abszissenachse |
| 94 96 | Handhabe Bilderfassungsoptikeinheit | 136 | Maximalwert der Abszissenachse |
| 98 | Strahlteiler | 138 | Ordinatenachse |
| 100 | Eingangsstrahlengang | 140 | Minimalwert der Ordinatenachse |
| 102 | Optik | 142 | Maximalwert der Ordinatenachse |
| 104 | Strahlengang | | |
| 106 | Strahlengang | 144 | Haupterstreckungsebene der weiteren Bilderfassungssensorik |
| 108 | Haupterstreckungsebene des Spektralfilters | 146 | Haupterstreckungsebene des weiteren Spektralfilters |
| 112 | Haupterstreckungsebene der Bilderfassungssensorik | 148 | Peak |
| | | 160 | Diagramm |
| | | 162 | Abszissenachse |
| 116 | Diagramm | 164 | Minimalwert der Abszissenachse |
| 118 | Abszissenachse | | |
| 120 | Minimalwert der Abszissenachse | 166 | Maximalwert der Abszissenachse |
| 168 | Ordinatenachse | 198 | Weitere zweite Zentralwellenlänge |
| 170 | Minimalwert der Ordinatenachse | 200 | Weitere zweite Filterkante |
| 172 | Maximalwert der Ordinatenachse | 202 | Weitere zweite Bandbreite |
| 174 | Weitere erste Filterkante | 204 | Weiteres erstes Beleuchtungsspektrum |
| 176 | Weitere erste Zentralwellenlänge | 206 | Peak |
| 178 | Weitere weitere erste Filterkante | 208 | Weiteres erstes Absorptionsspektrum |
| 180 | Weitere erste Bandbreite | 210 | Weiteres zweites Beleuchtungsspektrum |
| 182 | Diagramm | 212 | Peak |
| 184 | Abszissenachse | 214 | Weiteres zweites Absorptionsspektrum |
| 186 | Minimalwert der Abszissenachse | 218 | Verfahrensschritt |
| 188 | Maximalwert der Abszissenachse | 220 | Verfahrensteilschritt |
| 190 | Ordinatenachse | 222 | Verfahrensteilschritt |
| 192 | Minimalwert der Ordinatenachse | 224 | Verfahrensteilschritt |
| | | 226 | Verfahrensteilschritt |
| 194 | Maximalwert der Ordinatenachse | 228 | Bildgebungsfunktion |
| 196 | Weitere zweite Filterkante | 230 | Verfahrensschritt |

## Patentansprüche

1. Medizinische Bildgebungsvorrichtung, insbesondere endoskopische Bildgebungsvorrichtung, mit wenigstens einer Bilderfassungseinheit (10), welche wenigstens eine Bilderfassungssensorik (12) und wenigstens eine optische Filtereinheit (16) aufweist, welche wenigstens einen Spektralfilter (18) umfasst, welcher der Bilderfassungssensorik (12) zugeordnet und in Blickrichtung (20) der Bilderfassungssensorik (12) betrachtet vor dieser angeordnet ist, **dadurch gekennzeichnet, dass** der Spektralfilter (18) in einem ersten Betriebszustand dazu ausgebildet ist, Licht gemäß eines ersten spektralen Transmissionsbereichs (22) zur Bilderfassungssensorik (12) zu transmittieren und in einem zweiten Betriebszustand dazu ausgebildet ist, Licht gemäß eines zweiten spektralen Transmissionsbereichs (24), welcher von dem ersten spektralen Transmissionsbereich (22) zumindest teilweise verschieden ist, zur Bilderfassungssensorik (12) zu transmittieren.

2. Medizinische Bildgebungsvorrichtung nach Anspruch 1, **dadurch gekennzeichnet, dass** der erste Betriebszustand definiert ist als eine erste Stellung des Spektralfilters (18) relativ zur Bilderfassungssensorik (12) und/oder der zweite Betriebszustand definiert ist als eine zweite Stellung des Spektralfilters (18) relativ zur Bilderfassungssensorik (12).

3. Medizinische Bildgebungsvorrichtung nach Anspruch 1 oder 2, **dadurch gekennzeichnet, dass** der erste spektrale Transmissionsbereich (22) eine erste Filterkante (26) und der zweite spektrale Transmissionsbereich (24) eine zweite Filterkante (28) aufweist, welche relativ zur ersten Filterkante (26) spektral verschoben ist.

4. Medizinische Bildgebungsvorrichtung nach einem der vorhergehenden Ansprüche, **dadurch gekennzeichnet, dass** der erste spektrale Transmissionsbereich (22) eine erste Zentralwellenlänge (30) und der zweite spektrale Transmissionsbereich (24) eine zweite Zentralwellenlänge (32) aufweist, welche relativ zur ersten Zentralwellenlänge (30) spektral verschoben ist.

5. Medizinische Bildgebungsvorrichtung nach einem der vorhergehenden Ansprüche, **dadurch gekennzeichnet, dass** der erste spektrale Transmissionsbereich (22) eine erste Bandbreite (34) und der zweite spektrale Transmissionsbereich (24) eine zweite Bandbreite (36) aufweist, welche zumindest zu einem Großteil der ersten Bandbreite (34) entspricht.

6. Medizinische Bildgebungsvorrichtung nach einem der vorhergehenden Ansprüche, **dadurch gekennzeichnet, dass** der Spektralfilter (18) als ein Interferenzfilter (38) ausgebildet ist.

7. Medizinische Bildgebungsvorrichtung nach einem der vorhergehenden Ansprüche, **dadurch gekennzeichnet, dass** die Bilderfassungseinheit (10) dazu eingerichtet ist, den Spektralfilter (18) von dem ersten Betriebszustand in den zweiten Betriebszustand und/oder umgekehrt zu überführen.

8. Medizinische Bildgebungsvorrichtung nach einem der vorhergehenden Ansprüche, **dadurch gekennzeichnet, dass** die Bilderfassungseinheit (10) wenigstens ein, insbesondere als Kipp- oder Drehlager ausgebildetes Lager (40) umfasst, welches dazu ausgebildet ist, den Spektralfilter (18) von dem ersten Betriebszustand in den zweiten Betriebszustand und/oder umgekehrt überführbar zu lagern.

9. Medizinische Bildgebungsvorrichtung nach Anspruch 8, **dadurch gekennzeichnet, dass** die Bilderfassungseinheit wenigstens einen Aktor (42) umfasst, welcher dazu ausgebildet ist, den Spektralfilter (18) von dem ersten Betriebszustand in den zweiten Betriebszustand und/oder umgekehrt zu überführen.

10. Medizinische Bildgebungsvorrichtung nach Anspruch 9, **dadurch gekennzeichnet, dass** der Aktor (42), dazu ausgebildet ist, mindestens 15 mal pro Sekunde den Spektralfilter (18) von dem ersten Betriebszustand in den zweiten Betriebszustand des Spektralfilters und/oder umgekehrt zu überführen.

11. Medizinische Bildgebungsvorrichtung nach einem der vorhergehenden Ansprüche, **gekennzeichnet durch** wenigstens eine Beleuchtungseinheit (44), welche zur Beleuchtung eines von der Bilderfassungseinheit (10) zu erfassenden Objekts ausgebildet ist.

12. Medizinische Bildgebungsvorrichtung nach Anspruch 11, **dadurch gekennzeichnet, dass** die Beleuchtungseinheit (44) dazu ausgebildet ist, wenigstens ein erstes Beleuchtungslicht gemäß eines Beleuchtungsspektrums (46), welches zumindest zu einem Großteil außerhalb des ersten spektralen Transmissionsbereichs (22) liegt, bereitzustellen.

13. Medizinische Bildgebungsvorrichtung nach Anspruch 12, **dadurch gekennzeichnet, dass** das Beleuchtungsspektrum (46) zumindest zu einem Großteil einem Absorptionsspektrum (48) eines spontan Licht emittierenden Materials des zu beleuchtenden Objekts entspricht.

14. Medizinische Bildgebungsvorrichtung nach einem der Ansprüche 11 bis 13, **dadurch gekennzeichnet, dass** ein Emissionsspektrum (52) eines mittels der Beleuchtungseinheit (44) beleuchteten spontan Licht emittierenden Materials zumindest zu einem Großteil im ersten spektralen Transmissionsbereich (22) liegt.

15. Medizinische Bildgebungsvorrichtung nach einem der Ansprüche 10 bis14, **dadurch gekennzeichnet, dass** die Beleuchtungseinheit (44) dazu ausgebildet ist, wenigstens ein Beleuchtungslicht mit einem Beleuchtungsspektrum (46), insbesondere Weißlicht, bereitzustellen, welches zumindest zu einem Großteil in dem zweiten spektralen Transmissionsbereich (24) liegt.

16. Medizinische Bildgebungsvorrichtung nach einem der Ansprüche 10 bis 15, **dadurch gekennzeichnet, dass** ein weiteres Emissionsspektrum (54) eines mittels der Beleuchtungseinheit (44) beleuchteten weiteren spontan Licht emittierenden Materials zumindest zu einem Großteil außerhalb eines ersten spektralen Transmissionsbereichs (56) liegt.

17. Medizinische Bildgebungsvorrichtung nach einem der Ansprüche 10 bis 16, **dadurch gekennzeichnet, dass** ein weiteres Emissionsspektrum (58) eines mittels der Beleuchtungseinheit (44) beleuchteten weiteren spontan Licht emittierenden Materials zumindest zu einem Großteil innerhalb des zweiten spektralen Transmissionsbereichs (60) liegt.

18. Medizinische Bildgebungsvorrichtung nach einem der vorhergehenden Ansprüche, **dadurch gekennzeichnet, dass** die Filtereinheit (16) wenigstens eine schaltbare Abbildungslinse (58, 59) umfasst, welche zwischen Spektralfilter (18, 64) und Bilderfassungssensorik (12, 62) angeordnet ist und welche dazu ausgebildet ist, abhängig von einem Betriebszustand des Spektralfilters (18, 64), ein von dem Spektralfilter (18, 64) transmittiertes Transmissionsspektrum auf die Bilderfassungssensorik (12, 62) abzubilden.

19. Medizinische Bildgebungsvorrichtung nach einem der Ansprüche 10 bis 17, **dadurch gekennzeichnet, dass** die Filtereinheit (16) zumindest einen weiteren Spektralfilter (64) aufweist, welcher dazu ausgebildet ist, in einem weiteren ersten Betriebszustand Licht gemäß eines weiteren ersten spektralen Transmissionsbereichs (56) zu transmittieren und in einem weiteren zweiten Betriebszustand dazu ausgebildet ist, Licht gemäß eines weiteren zweiten spektralen Transmissionsbereichs (60) zu transmittieren.

20. Medizinische Bildgebungsvorrichtung nach Anspruch 18, **dadurch gekennzeichnet, dass** zumindest einer der weiteren Transmissionsbereiche (56, 60) des weiteren Spektralfilters (64) von zumindest einem Transmissionsbereich (22, 24) der Transmissionsbereiche (22, 24) des weiteren Spektralfilters (64)verschieden ist.

21. Medizinische Bildgebungsvorrichtung nach Anspruch 18 oder 19, **dadurch gekennzeichnet, dass** die Bilderfassungseinheit (10) zumindest eine weitere Bilderfassungssensorik (13) umfasst, welche dem weiteren Spektralfilter (64) zugeordnet und in Blickrichtung (66) der weiteren Bilderfassungssensorik (13) betrachtet vor diesem angeordnet ist.

22. Medizinische Bildgebungsvorrichtung nach einem der vorhergehenden Ansprüche, **gekennzeichnet durch** wenigstens eine Steuereinheit (68), welche dazu ausgebildet ist, zumindest die Bilderfassungseinheit (10) anzusteuern.

23. Medizinische Bildgebungsvorrichtung nach Anspruch 22, **dadurch gekennzeichnet, dass** die Steuereinheit (68) dazu ausgebildet ist, in Abhängigkeit von wenigstens eines Betriebszustands wenigstens eines Spektralfilters (18, 64) der Filtereinheit (16) zumindest eine Bilderfassungssensorik (12) zu aktivieren oder zu deaktivieren.

24. Medizinische Bildgebungsvorrichtung nach Anspruch 22 oder 23, **dadurch gekennzeichnet, dass** die Steuereinheit (68) dazu ausgebildet ist, in Abhängigkeit von wenigstens einem Betriebszustand wenigstens eines Spektralfilters (18, 64) der Filtereinheit (16) zumindest ein Beleuchtungslicht gemäß wenigstens eines Beleuchtungsspektrums der Beleuchtungseinheit (44) zu aktivieren oder zu deaktivieren.

25. Medizinische Bildgebungsvorrichtung nach einem der Ansprüche 22 bis 24, **dadurch gekennzeichnet, dass** die Steuereinheit (68) dazu ausgebildet ist, in Abhängigkeit von wenigstens einem Betriebszustand wenigstens eines Spektralfilters (18, 64) einen Schaltzustand der schaltbaren Abbildungslinse (58, 59) zu aktivieren oder zu deaktivieren.

26. Medizinische Bildgebungsvorrichtung nach einem der vorhergehenden Ansprüche, **dadurch gekennzeichnet, dass** diese zumindest teilweise ein medizinisches Bildgebungsgerät, insbesondere ein endoskopisches Bildgebungsgerät, ausbildet.

27. Verfahren zum Betrieb einer medizinischen Bildgebungsvorrichtung, insbesondere einer endoskopischen Bildgebungsvorrichtung, vorzugsweise nach einem der Ansprüche 1 bis 26, bei welchem wenigstens ein Spektralfilter (18) einer optischen Filtereinheit (16) einer Bilderfassungssensorik (12) zugeordnet und in Blickrichtung (20) der Bilderfassungssensorik (12) betrachtet vor dieser angeordnet ist, **dadurch gekennzeichnet, dass** in wenigstens einem Verfahrensschritt der Spektralfilter (18) in wenigstens einem ersten Betriebszustand Licht gemäß eines ersten spektralen Transmissionsbereichs (22) zur Bilderfassungssensorik (12) transmittiert und in einem weiteren Verfahrensschritt der Spektralfilter (18) in wenigstens einem zweiten Betriebszustands Licht gemäß eines zweiten spektralen Transmissionsbereichs (24), welcher von dem ersten spektralen Transmissionsbereich (22) zumindest teilweise verschieden ist, zur Bilderfassungssensorik (12) transmittiert.
